# EUROPEAN PATENT SPECIFICATION

(11) **EP 4 215 064 B1**
(45) Date of publication and mention of the grant of the patent: **14.01.2026**
(21) Application number: 22879585.2
(22) Date of filing: 17.11.2022
(51) Int. Cl.: A24B 15/30, A24B 15/34, A24B 15/32, A24B 15/40, A24D 1/00, A23L 27/20, A24B 15/16, C07C 69/96

(54) **NOVEL FLAVOR, FLAVOR COMPOSITION, AND PRODUCT INCLUDING SAME**
NEUARTIGER GESCHMACK, AROMAZUSAMMENSETZUNG UND PRODUKT DAMIT
NOUVEL ARÔME, COMPOSITION AROMATIQUE ET PRODUIT LE COMPRENANT

(30) Priority: 18.11.2021 KR 20210159812
(43) Date of publication of application: 26.07.2023
(73) Proprietor: KT & G Corporation, Daejeon 34337 (KR)
(72) Inventor: LEE, Changgook, Daejeon 34128 (KR); JUNG, Kyung Bin, Daejeon 34128 (KR); KIM, Dong Hyun, Daejeon 34128 (KR); KIM, Ick Joong, Daejeon 34128 (KR); SONG, In Beom, Daejeon 34128 (KR); SONG, Ho Rim, Daejeon 34128 (KR); WOO, Ji Seob, Daejeon 34128 (KR); LEE, Geon Chang, Daejeon 34128 (KR)
(74) Representative: Ter Meer Steinmeister & Partner
(86) International application number: PCT/KR2022/018181
(87) International publication number: WO 2023/090889

(56) References cited:
- EP-A1- 0 510 817
- CN-A- 102 311 464
- CN-A- 102 311 465
- KR-A- 20110 106 221
- US-A- 4 002 179
- US-A- 4 509 537
- US-A- 5 703 123
- US-A1- 2002 193 269

## Description

### Technical Field

The following description relates to a novel flavoring agent in which flavor ingredients are released by heat, a flavoring agent composition, and a product including the same.

### Background Art

Flavoring agents may be added to food and smoking articles to further improve the taste. Smoking articles are manufactured so that smoke or aerosol generated in the smoking articles moves from upstream to downstream and is delivered to the smoker to feel the smoking satisfaction. There are various factors that determine smoking satisfaction, but the most important is the cigarette taste that the smoker feels. A smoker wants to enjoy a variety of cigarette tastes in one smoking article, and cigarette manufacturers add flavoring substances (e.g., flavoring agents) to satisfy such a desire of the smoker so that the smoker may feel various flavors or savors.

Existing flavoring agents are highly likely to decompose the chemical structure at room temperature when the smoking medium is stored for a long period of time, and it is difficult to express sufficient flavor capable of enhancing the cigarette taste during smoking due to volatilization of flavor ingredients, or persistence of flavor is weak or cigarette taste is changed as smoking time elapses. Accordingly, it is necessary to develop a flavoring agent capable of increasing smoking satisfaction during smoking. Flavoring agents are applied to add various flavors to foods, but when foods are processed and/or stored for a long time, the flavor is frequently volatilized and released and disappeared. Therefore, it is necessary to develop a flavoring agent which is capable of preventing or delaying the release of volatile flavor to increase storage life and is capable of sufficiently expressing flavor when used by consumers. US 4 509 537 A relates to providing smoking compositions which contain a monocarbonate ester compound as a flavorant additive. EP 0 510 817 A1 relates to providing smoking compositions which contain a vanillin flavorant-release additive. US 4 002 179 A relates to oligomers and polymers produced from monomer carbonate esters of menthol. US 2002/193269 A1 relates to a group of compounds having protected hydroxy groups which are precursors for organoleptic compounds, such as fragrances and masking agents and antimicrobial compounds. KR 2011 0106221 A relates to a sidestream smoke-reducing cigarette capable of reducing sidestream smoke, a method for producing a cigarette paper capable of reducing sidestream smoke and preventing a reduction in taste of tobacco smoke, and a sidestream smoke-reducing cigarette prepared using the method. CN 102 311 464 A relates to a class of monosaccharide ester cigarette humectants, in particular to monomenthoxycarbonyl monosaccharide ester compounds, a preparation method thereof, and an application thereof as a cigarette humectant having humectant properties and both flavor enhancement and slow-release flavoring effects. CN 102 311 465 A relates to a type of monosaccharide ester cigarette humectant, and specifically to monosaccharide geraniol carbonate monoester compounds, a preparation method thereof, and an application thereof as cigarette humectant having humectant properties and both flavor enhancement and slow-release flavoring effects. US 5 703 123 A relates to agents without a troublesome characteristic smell and characteristic taste which, when applied to the skin or to the mucosae, cause a physiological cooling effect.

### Disclosure of the Invention

### Technical Goals

Existing compounds having a flavoring agent function have low chemical structural stability at room temperature (rt) or a temperature close thereto so that structural transformation or decomposition may occur, and thus flavor ingredients may volatilize. In order to solve this problem, an objective to be achieved by the present disclosure is to provide a novel flavoring agent in which flavor ingredients are released by thermal decomposition when heat is applied.

The present disclosure relates to a flavoring agent composition including the novel flavoring agent according to the present disclosure.

The present disclosure relates to a product including the novel flavoring agent according to the present disclosure.

However, the problem to be solved by the present disclosure is not limited to those mentioned above, and other problems not mentioned will be clearly understood by those skilled in the art from the description below.

### Technical Solutions

According to one embodiment of the present disclosure, the present disclosure relates to a flavoring agent, which is a compound represented by Formula 1 below. 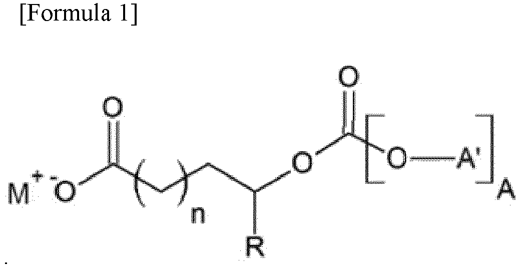 (In Formula 1,
n is an integer of 1 or 2,
M is selected from alkali metals and transition metals,
R is a straight-chain or branched-chain alkyl group having 1 to 30 carbon atoms, and
moiety A is a moiety derived from a flavoring compound having at least one of an aromatic ring, an aliphatic ring, and an aliphatic chain which have a hydroxyl group, in which the hydroxyl group participates in a carbonate linking group ( 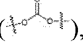 ), and A' corresponds to a flavoring compound except for the hydroxyl group.)

According to one embodiment of the present disclosure, the flavoring compound may be selected from a cyclic monoterpene-based compound having a hydroxyl group, a monoterpene-based acyclic compound having a hydroxyl group, a C₆-C₁₀ aromatic compound having a hydroxyl group, and a C₅-C₆ non-aromatic ring having a hydroxyl group.

According to one embodiment of the present disclosure, the transition metal may be selected from Zr, Mg, Ca, Co, Rh, Ir, Nb, Pd, Pt, Fe, Ru, Os, Cr, Mo, W, Mn, Tc, Re, Cu, Ag, and Au, and the alkali metal may be selected from Li, Na, K, Rb, and Cs.

According to one embodiment of the present disclosure, the flavoring agent may be a flavoring agent compound that develops flavor upon thermal decomposition.

According to one embodiment of the present disclosure, the flavoring agent may be decomposed into the flavoring compound, a lactone compound, and carbon dioxide during thermal decomposition.

According to one embodiment of the present disclosure, the compound may be thermally decomposed at a temperature of 80°C or higher.

According to one embodiment of the present disclosure, the flavoring agent may be a flavoring agent for food or smoking articles.

According to one embodiment of the present disclosure, the present disclosure relates to a composition including a flavoring agent according to the present disclosure.

According to one embodiment of the present disclosure, the composition may be a solid phase, a slurry, a paste, a gel, a liquid phase, an emulsion, or an aerosol.

According to one embodiment of the present disclosure, the composition may further include a carrier, or an additive acceptable for food or smoking articles, or both thereof.

According to one embodiment of the present disclosure, the present disclosure relates to a smoking article including a flavoring agent according to the present disclosure.

According to one embodiment of the present disclosure, the smoking article may include a slurry, a paste, a liquid phase, a gel, a powder, beads, a sheet, a film, a fiber, or a molded body containing the flavoring agent.

According to one embodiment of the present disclosure, the smoking article may be a cigarette or an electronic cigarette.

According to one embodiment of the present disclosure, the present disclosure relates to a food containing a flavoring agent according to the present disclosure.

According to one embodiment of the present disclosure, the food may be mixed or cooked by heat with the flavoring agent according to the present disclosure.

### Effects

According to one embodiment of the present disclosure, when the flavoring agent according to the present disclosure is applied to a smoking article, since flavor ingredients are developed during smoking to relieve the acrid smell of sidestream smoke, and the flavor ingredients are released during thermal decomposition by heating, so that the taste of cigarette can be improved, and the taste of cigarette can be constantly maintained.

According to one embodiment of the present disclosure, since the flavoring agent according to the present disclosure is thermally decomposed by heating to release the flavor ingredients, when applied to food, a rich flavor during the cooking process can be provided, and the storage life of the flavoring agent during the storage process of food can be extended.

### Brief Description of Drawings

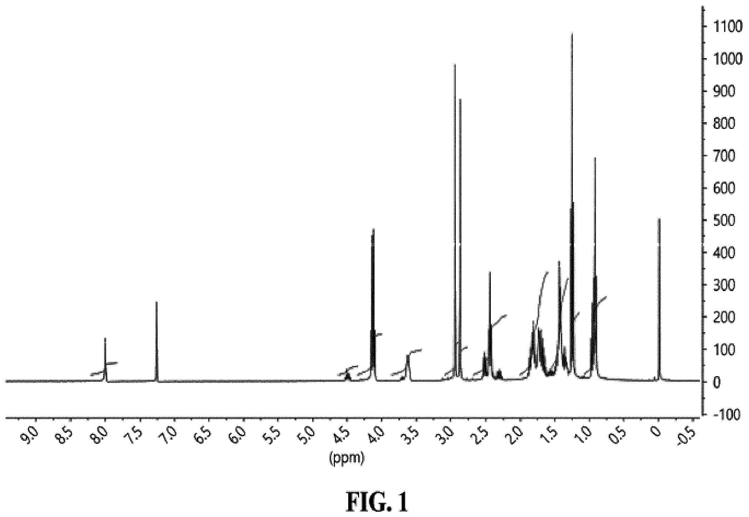

FIG. 1 is a diagram illustrating the results of NMR analysis of ethyl 4-hydroxyheptanoate (2a) prepared in an example according to one embodiment of the present disclosure.

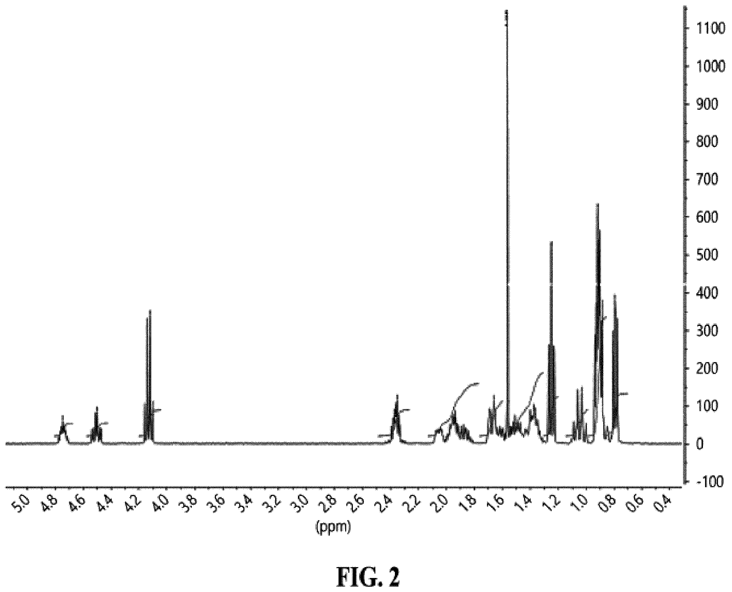

FIG. 2 is a diagram illustrating the results of NMR analysis of ethyl 4-(mentylcarbonyloxy)heptanoate (3a) prepared in an example according to one embodiment of the present disclosure.

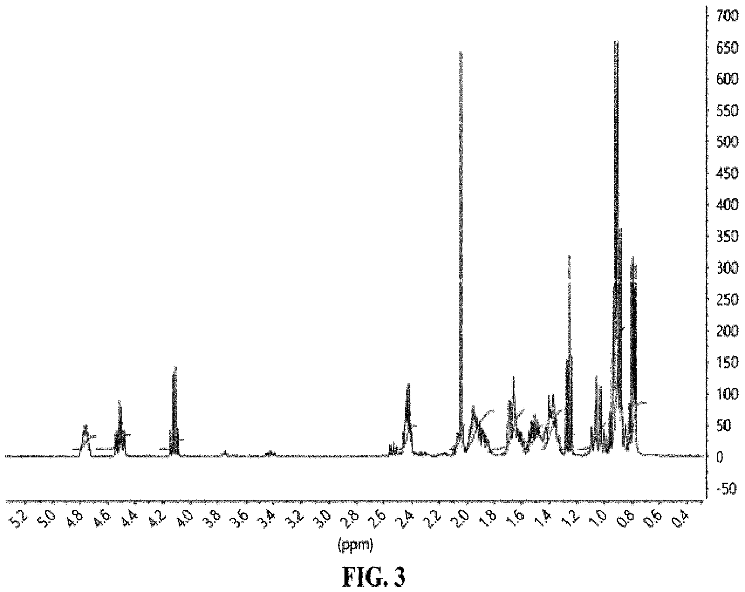

FIG. 3 is a diagram illustrating the results of NMR analysis of 4-(mentylcarbonyloxy)heptanoic acid (4a) prepared in an example according to one embodiment of the present disclosure.

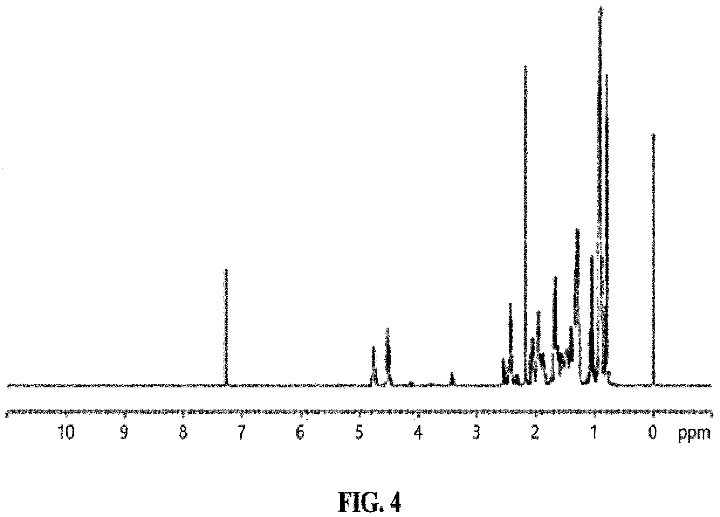

FIG. 4 is a diagram illustrating the results of NMR analysis of 4-(mentylcarbonyloxy)nonanoic acid (4b) prepared in an example according to one embodiment of the present disclosure.

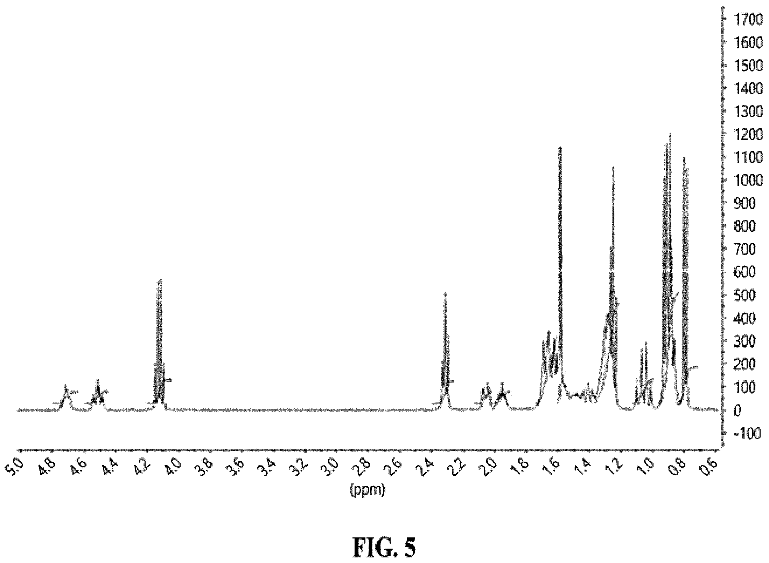

FIG. 5 is a diagram illustrating the results of NMR analysis of 5-(methylcarbonyloxy)decanoate (3c) prepared in an example according to one embodiment of the present disclosure.

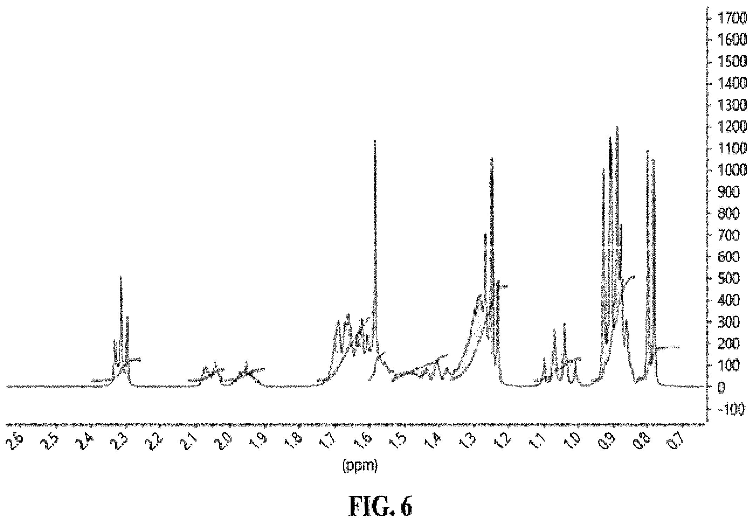

FIG. 6 is a diagram illustrating the results of NMR analysis of 5-(mentylcarbonyloxy)decanoate (3c) prepared in an example according to one embodiment of the present disclosure.

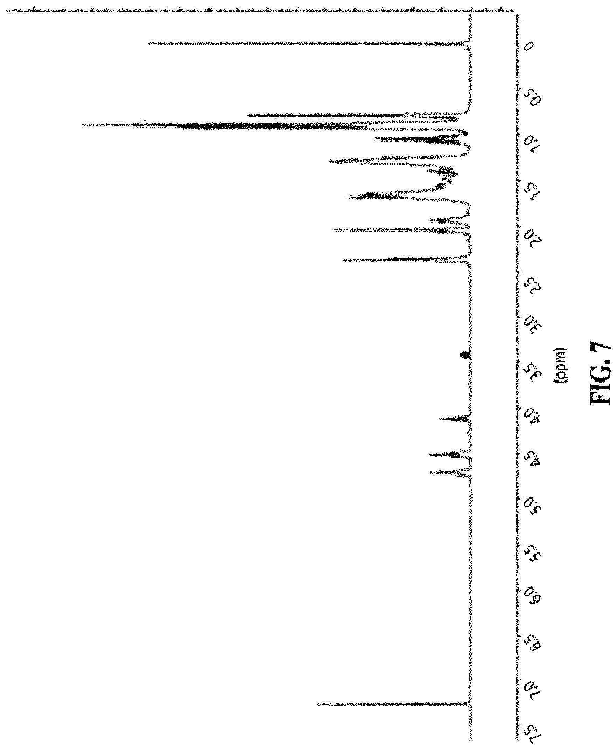

FIG. 7 is a diagram illustrating the results of NMR analysis of 5-(mentylcarbonyloxy)decanoic acid (4c) prepared in an example according to one embodiment of the present disclosure.

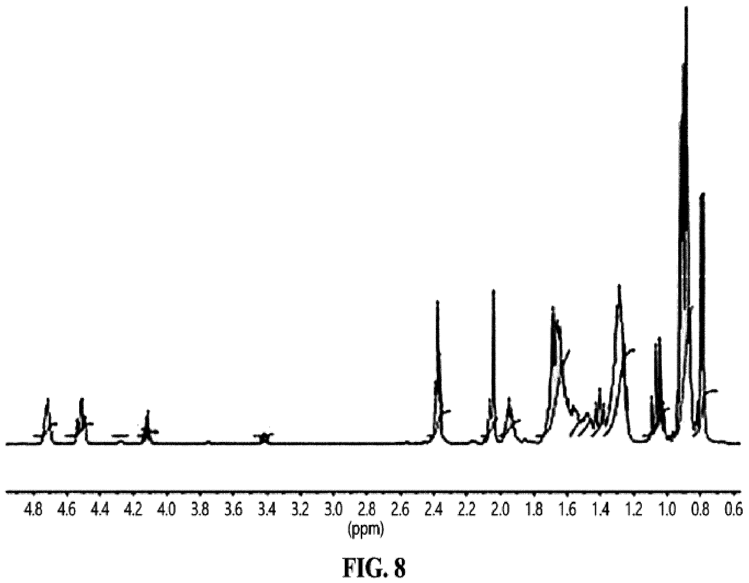

FIG. 8 is a diagram illustrating the results of NMR analysis of 5-(mentylcarbonyloxy)decanoic acid (4c) prepared in an example according to one embodiment of the present disclosure.

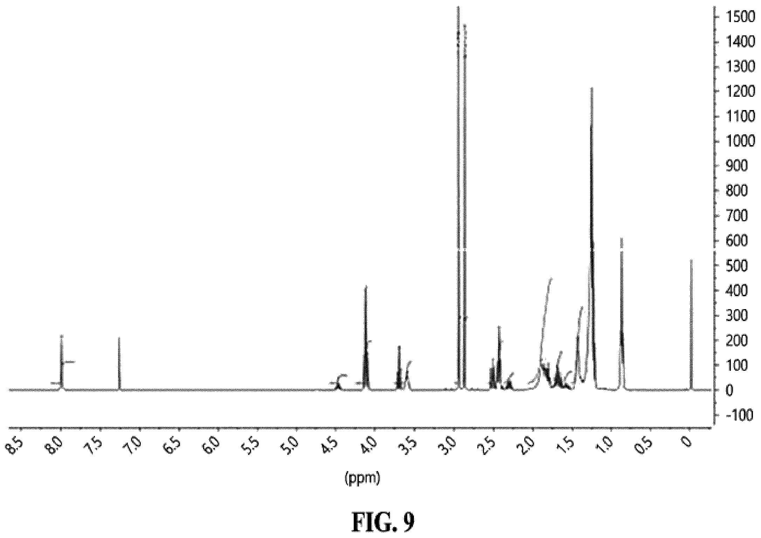

FIG. 9 is a diagram illustrating the results of NMR analysis of ethyl 4-hydroxyundecanoate (2d) prepared in an example according to one embodiment of the present disclosure.

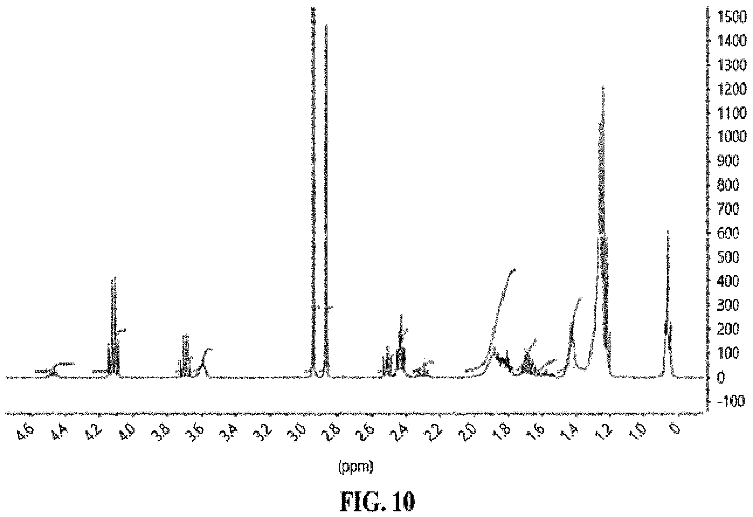

FIG. 10 is a diagram illustrating the results of NMR analysis of ethyl 4-hydroxyundecanoate (2d) prepared in an example according to one embodiment of the present disclosure.

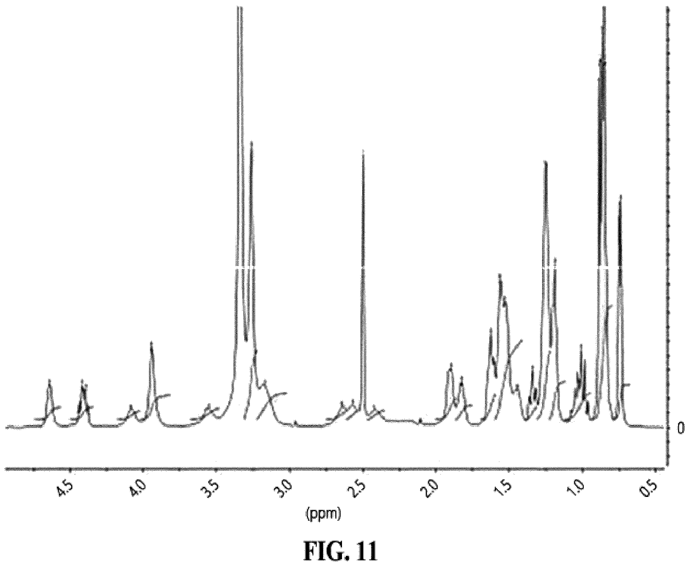

FIG. 11 is a diagram illustrating the results of NMR analysis of ethyl 4-(mentylcarbonyloxy)undecanoate (3d) prepared in an example according to one embodiment of the present disclosure.

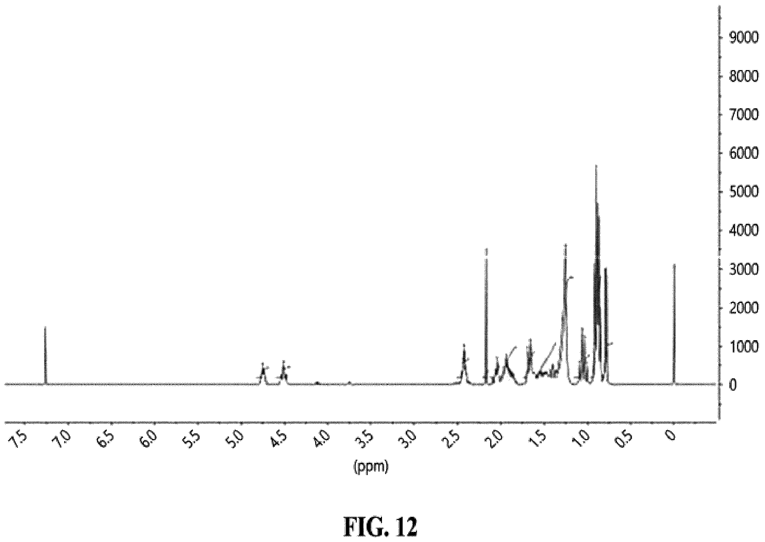

FIG. 12 is a diagram illustrating the results of NMR analysis of 4-(mentylcarbonyloxy)undecanoic acid (4d) prepared in an example according to one embodiment of the present disclosure.

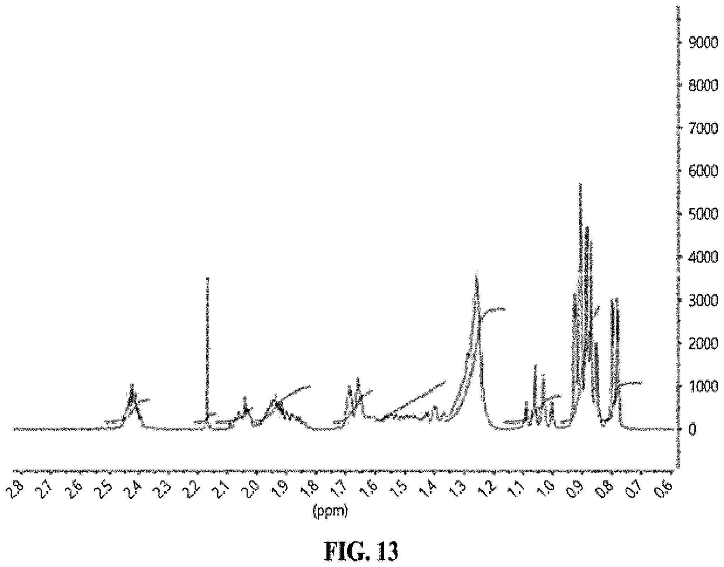

FIG. 13 is a diagram illustrating the results of NMR analysis of 4-(mentylcarbonyloxy)undecanoic acid (4d) prepared in an example according to one embodiment of the present disclosure.

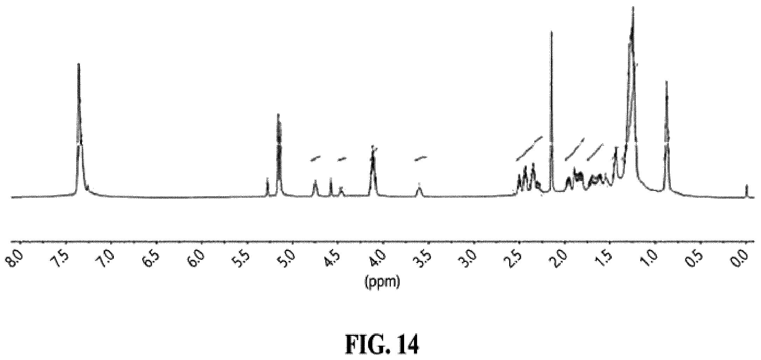

FIG. 14 is a diagram illustrating the results of NMR analysis of ethyl 4-(benzyloxycarbonyloxy)undecanoate (3e) prepared in an example according to one embodiment of the present disclosure.
FIG. 15 is a diagram illustrating the results of thermal analysis of sodium (4-mentylcarbonyloxy)undecanoate (5d) prepared in an example according to one embodiment of the present disclosure.
FIG. 16 is a diagram illustrating the distribution of components according to the thermal decomposition temperature of sodium (4- mentylcarbonyloxy)undecanoate (5d) prepared in an example according to one embodiment of the present disclosure.

### Best Mode for Carrying Out the Invention

Hereinafter, embodiments of the present disclosure will be described in detail with reference to the accompanying drawings. In describing the present disclosure, if it is determined that a detailed description of a related well-known function or configuration may unnecessarily obscure the gist of the present disclosure, the detailed description thereof will be omitted. Also, terms used in the present specification, as terms which are used so as to appropriately describe a preferred embodiment of the present disclosure, may be changed depending on the user's or operator's intention or the practices of the field to which the present disclosure pertains. Therefore, the definitions of the terms should be made based on the contents throughout the present specification. The same reference numerals disclosed in each drawing represent the same members.

Throughout the specification, when a member is said to be located "on" other member, this includes not only a case in which a member is in contact with other member but also a case in which another member exists between the two members.

Throughout the specification, when a part "includes" a certain component, it means that other components may be further included, rather than excluding other components.

Hereinafter, the novel flavoring agent according to the present disclosure and the use of the flavoring agent will be described in detail with reference to embodiments and drawings. However, the present disclosure is not limited to these embodiments and drawings.

The present disclosure relates to a novel flavoring agent, and according to one embodiment of the present disclosure, the flavoring agent may develop flavor ingredients by thermal decomposition when heat is applied.

According to one embodiment of the present disclosure, the flavoring agent may be a compound represented by Formula 1 below.

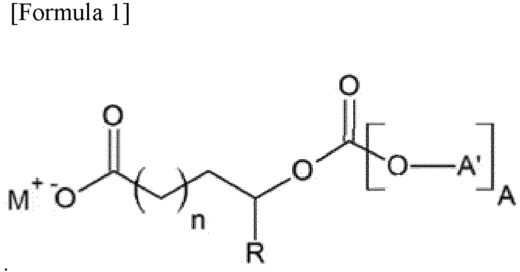

As an example of the present disclosure the flavoring compound in Formula 1 is covalently bonded with a carbonate linking group ( 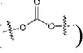 ), and when heat is applied, the compound of Formula 1 is thermally decomposed to be decomposed into a flavoring compound and a lactone compound so that flavor ingredients may be expressed. For example, the compound of Formula 1 reacts with a hydroxyl group of the flavoring compound through a ring opening mechanism of a lactone-based compound to covalently bond the flavoring compound through a carbonate linking group. It may act as a protecting group to prevent conversion to lactone compounds due to ring closure at room temperature (rt) and/or a temperature close thereto. The compound of Formula 1 has structural stability at about room temperature (rt) or a temperature close thereto, has low volatility, and receives heat to break the carbonate linking group with a ring closing mechanism so that the compound of Formula 1 may be decomposed into a lactone-based compound and a flavoring compound to develop flavor. Carbon dioxide which is harmless to the human body may be generated during the decomposition process. That is, the carbonate linking group is broken by heat so that the compound of Formula 1 is decomposed into flavoring compounds, and carbon dioxide may be generated. Next, due to ring closure, the compound of Formula 1 may be decomposed into lactone-based compounds to develop flavor.

According to one embodiment of the present disclosure, in Formula 1, n may be an integer of 1 or 2. R may be a straight-chain or branched-chain alkyl having 1 to 30 carbon atoms, and preferably a straight-chain or branched-chain alkyl having 2 to 10 carbon atoms.

According to one embodiment of the present disclosure, moiety A in Formula 1 may be a moiety derived from a flavoring compound having at least one of an aromatic ring having a hydroxyl group, an aliphatic ring having a hydroxyl group, and an aliphatic chain having a hydroxyl group. The hydroxyl group may include one or more (e.g., one or two) in a ring, a chain, or both thereof. This may correspond to a hydroxyl group-containing substituent, a basic backbone, and/or a moiety. The hydroxyl group participates in the carbonate linking group in Formula 1, and A' may correspond to a flavoring compound excluding the hydroxyl group. That is, since the hydroxyl group of the flavoring compound in moiety A is protected with a carbonate linking group, a decomposition reaction may be prevented by ring-closure at room temperature. According to one embodiment of the present disclosure, the flavoring compound may be selected from a cyclic monoterpene-based compound having a hydroxyl group, a monoterpene-based acyclic compound having a hydroxyl group, a C₆-C₁₀ aromatic compound having a hydroxyl group, a C₅-C₁₀ or C₅-C₆ non-aromatic ring having a hydroxyl group, and isomers thereof. For example, the flavoring compound may be selected from the formulas below and may be a compound which is produced when the carbonate linking group of Formula 1 is broken during thermal decomposition.

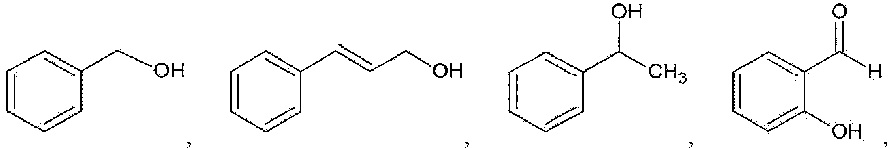

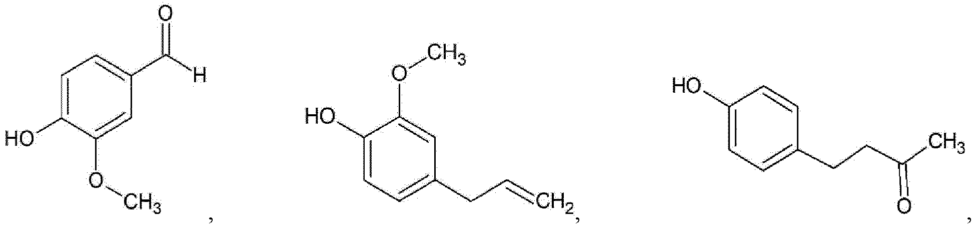

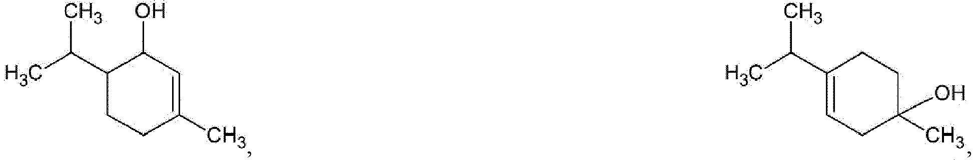

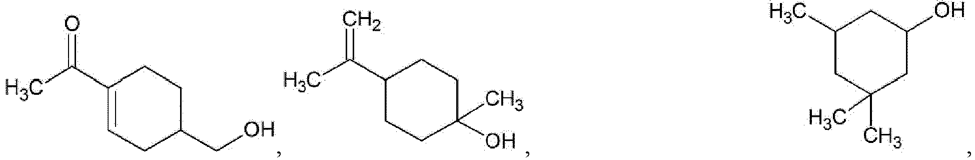

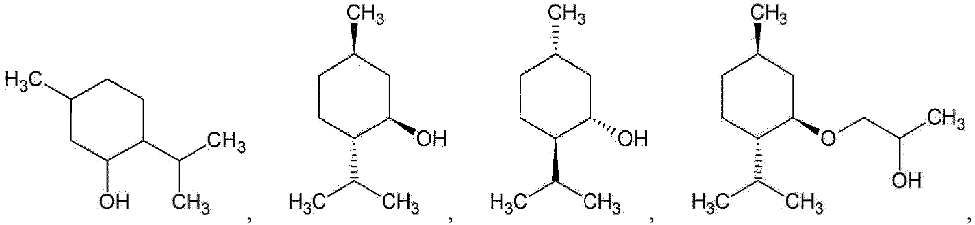

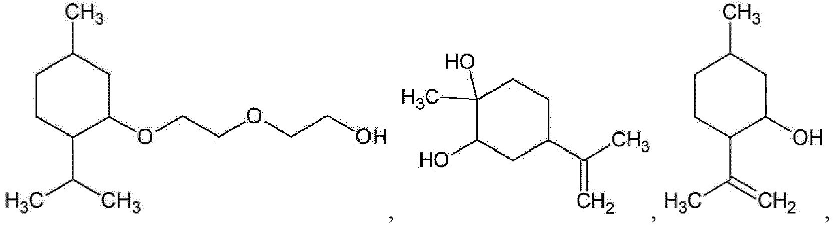

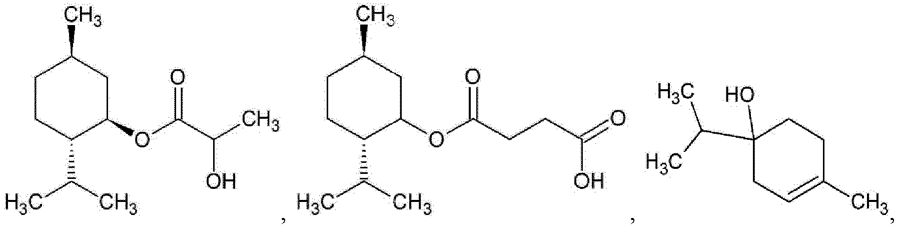

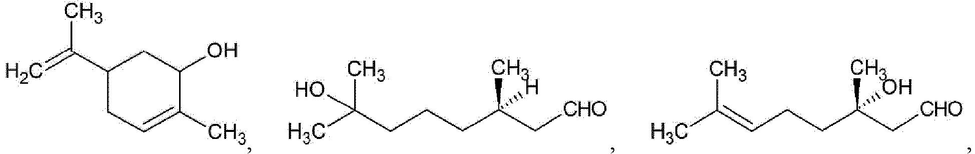

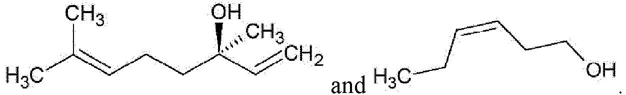

According to one embodiment of the present disclosure, A' in the moiety A may be selected from the formulas below. Here, * corresponds to the oxygen position in the carbonate linking group.

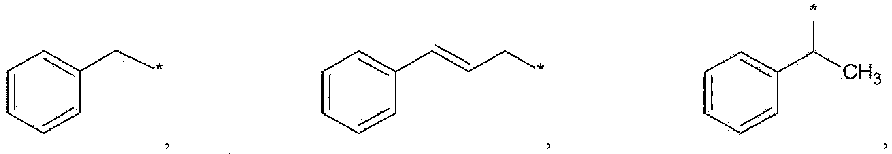

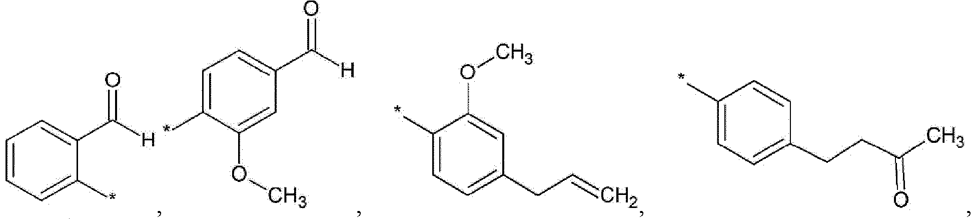

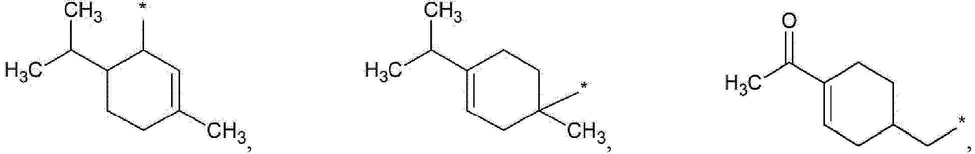

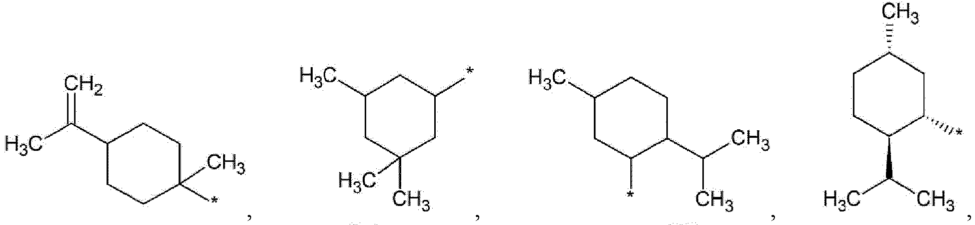

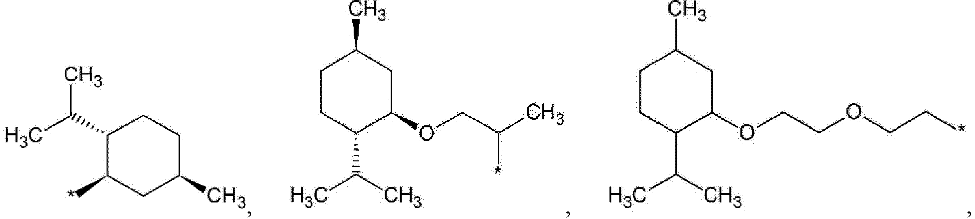

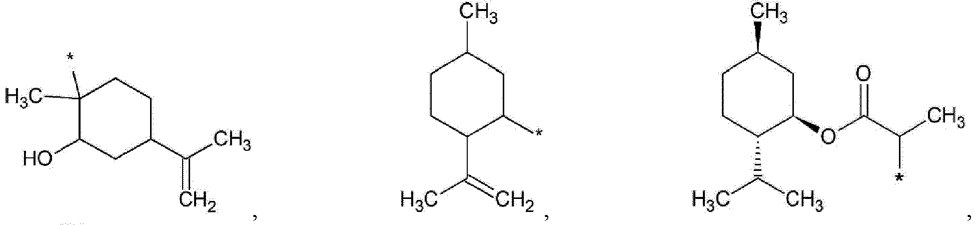

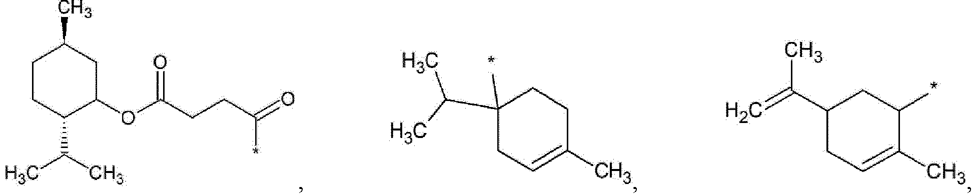

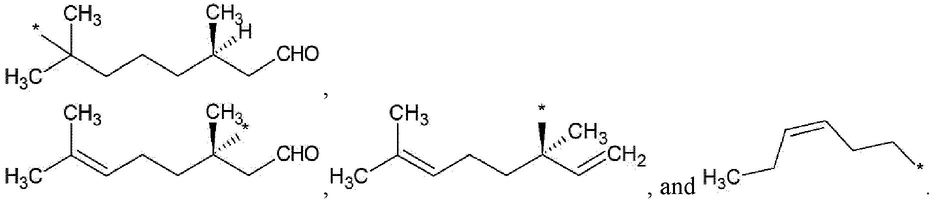

According to one embodiment of the present disclosure, M is selected from an alkali metal and a transition metal, and M may form a salt with oxygen of an ester group to increase solubility in water-soluble solvents, and to facilitate the application of food and smoking articles. For example, the transition metal may be selected from Zr, Mg, Ca, Co, Rh, Ir, Nb, Pd, Pt, Fe, Ru, Os, Cr, Mo, W, Mn, Tc, Re, Cu, Ag, and Au. For example, the alkali metal may be selected from Li, Na, K, Rb, and Cs. For example, M may be a metal that forms a monovalent cation and may be selected from Li, Na, and K.

According to one embodiment of the present disclosure, the lactone compound may be gamma lactone of Formula 2 below or delta lactone of Formula 3 below.

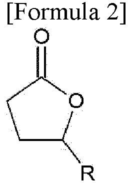

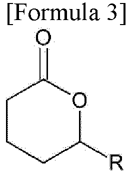

As an example of the present disclosure, R in Formulas 1 and 2 may be a straight-chain or branched-chain alkyl group having 1 to 30 carbon atoms, and preferably a straight-chain or branched-chain alkyl group having 2 to 10 carbon atoms.

According to one embodiment of the present disclosure, the lactone compound may be selected from the formulas below.

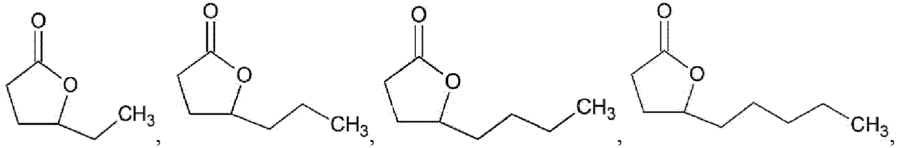

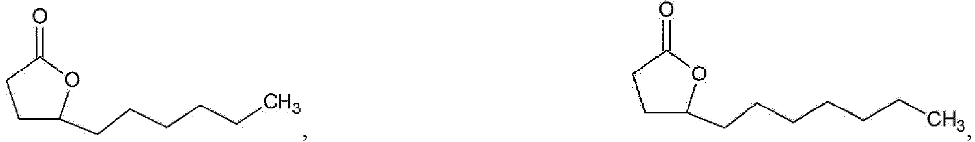

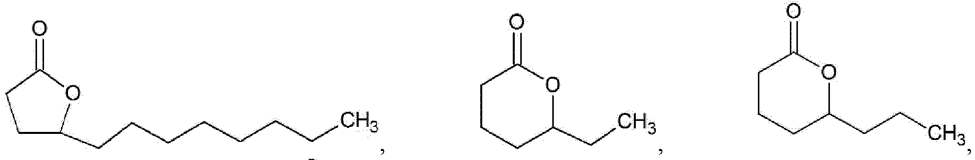

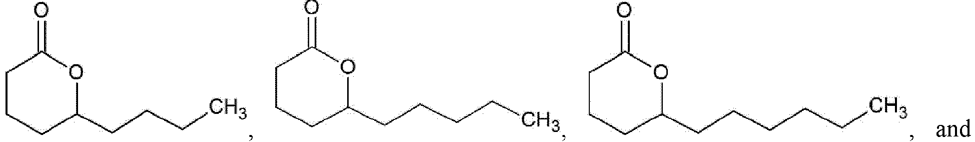

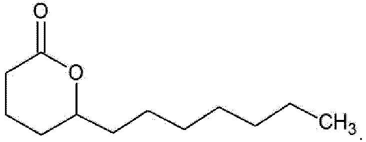

According to one embodiment of the present disclosure, the compound may be selected from Formulas 1-1 to 1-26 below.

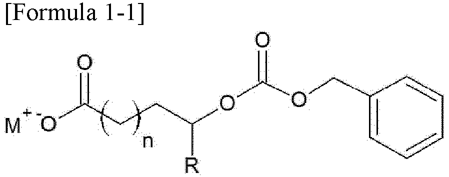

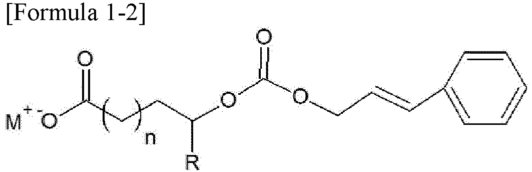

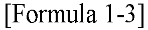

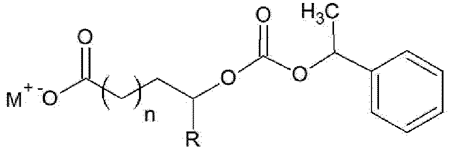

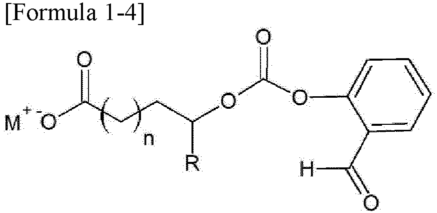

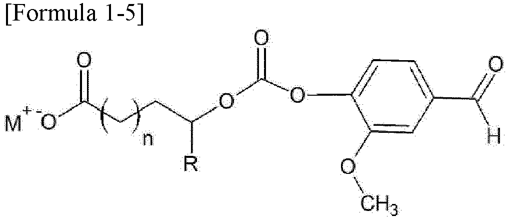

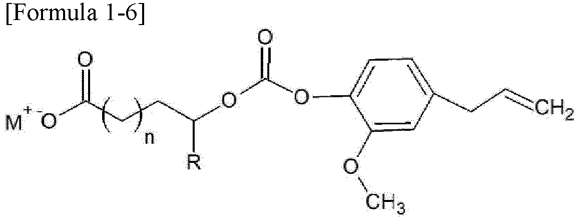

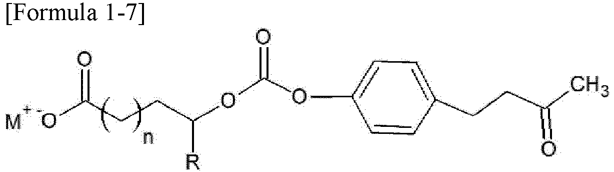

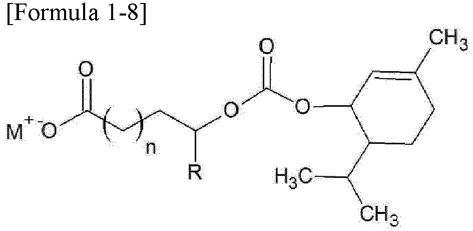

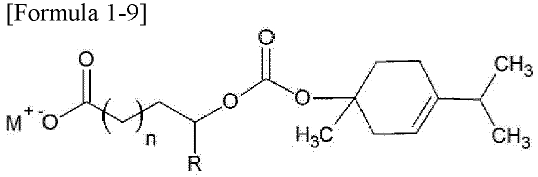

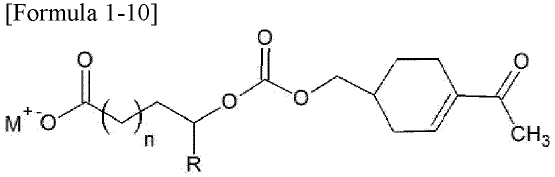

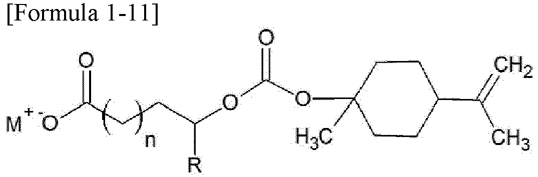

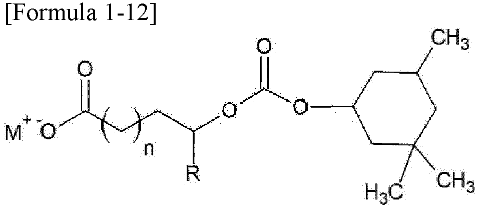

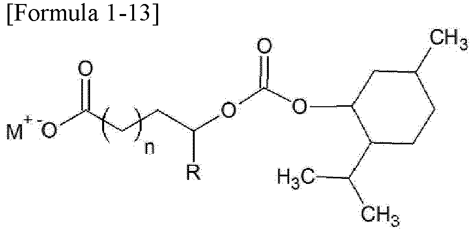

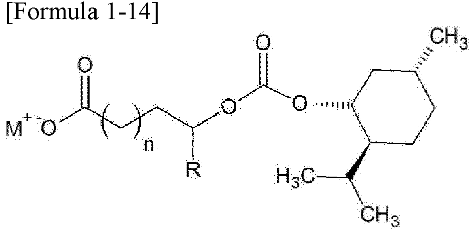

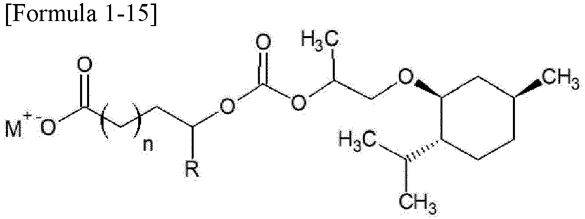

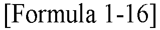

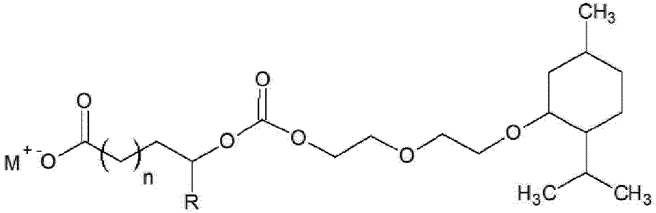

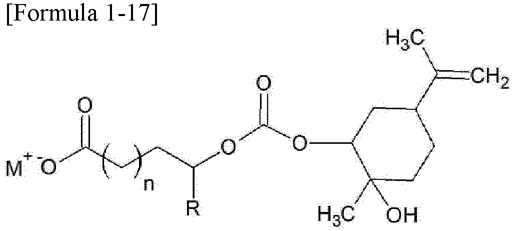

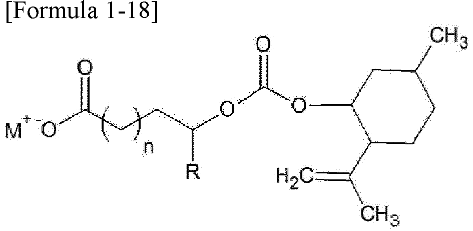

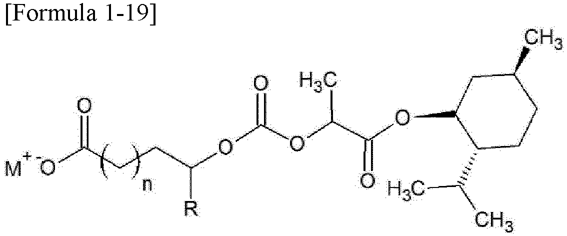

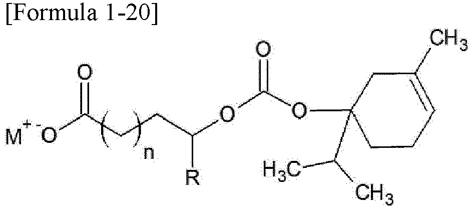

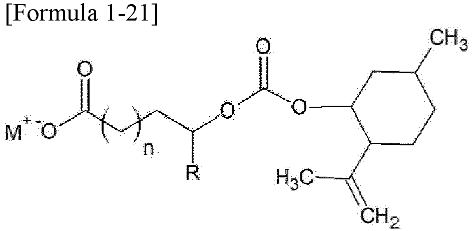

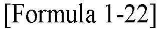

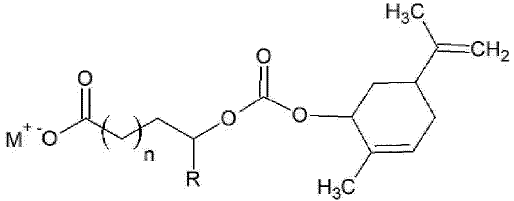

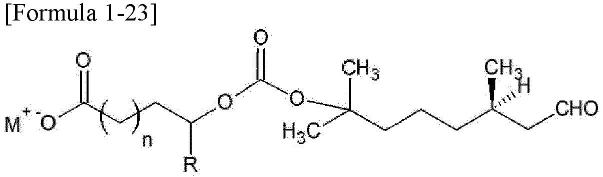

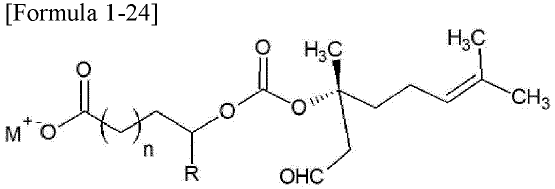

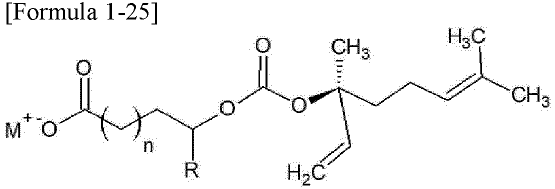

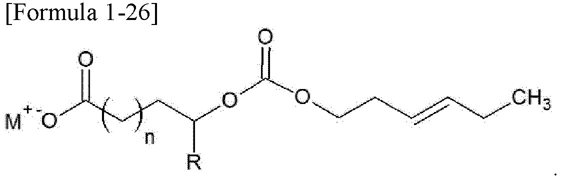

(Here, M and R are as defined in Formula 1 above.)

According to one embodiment of the present disclosure, the compound may be thermally decomposed at a temperature of 70°C or higher; 80°C or higher; 90°C or higher; or 100°C or higher, preferably 120°C or higher; 150°C or higher; or 200°C or higher, and more preferably 200°C to 300°C. In addition, the compound may be thermally decomposed in an environment containing oxygen and/or moisture.

According to one embodiment of the present disclosure, the flavoring agent may be used as a flavoring agent in food and smoking articles. That is, the flavoring agent may be used as an acceptable additive for food and smoking articles.

The present disclosure relates to a composition including a flavoring agent according to the present disclosure.

According to one embodiment of the present disclosure, the composition may include the flavoring agent (i.e., the flavoring compound represented by Formula 1) according to the present disclosure, and may further include carriers, additives, or both thereof depending on the use. The carriers and additives are acceptable carriers and additives for food or smoking articles, and may include, for example, solvents, binders, diluents, decomposing agents, lubricants, flavoring agents, colorants, preservatives, antioxidants, emulsifiers, stabilizers, flavor enhancers, and sweeteners, but are not limited thereto.

According to one embodiment of the present disclosure, the composition may further include a base matrix (or matrix) component depending on the use, and the base matrix component may be, for example, paper, pulp, wood, polymer resin (e.g., cellulose), fiber, vegetable oils, petroleum oils (e.g., paraffins), animal oils, waxes, fatty acids (e.g., animal fats with 1 to 50 carbon atoms, vegetable fats, saturated fatty acids, or unsaturated fatty acids (e.g., mono- or poly-unsaturated fatty acids)). Organic and/or inorganic or ceramic powders (e.g., chalk, perlite, vermiculite, diatomaceous earth, colloidal silica, magnesium oxide, magnesium sulfate sulfuric, and magnesium carbonate), wetting agents (e.g., glycerin or propylene glycol), acetate compounds, and the like, may be further added to the base matrix component.

According to one embodiment of the present disclosure, the composition may further include tobacco ingredients depending on the use. When the composition is applied to smoking articles, it is possible to develop flavors in mainstream smoke and sidestream smoke under smoking conditions. The tobacco ingredients may be solid materials based on tobacco raw materials such as sheet-shaped tobacco, cut tobacco, and reconstituted tobacco, and may be selected from leaf tobacco, extruded tobacco, and bandcast tobacco. In addition, the composition may further include an aerosol-generating agent applicable as a cigarette medium, and the aerosol-generating agent may be sorbitol, glycerol, propylene glycol, triethylene glycol, lactic acid, diacetin, triacetin, triethylene glycol diacetate, triethyl citrate, ethyl myristate, isopropyl myristate, methyl stearate, dimethyl dodecanedioate, dimethyl tetradecanedioate, and the like, but is not limited thereto.

According to one embodiment of the present disclosure, the flavoring agent may be contained in the composition in an amount of 0.0001 wt% or more; 0.001 wt% or more; 0.01 wt% or more; 0.0001 wt% to 100 wt% (or less than 100 wt%); 0.1 wt% to 80 wt%; 0.0001 wt% to 60 wt%; 0.001 wt% to 50 wt%; 0.1 wt% to 30 wt%; 1 wt% to 20 wt%; 5 wt% to 20 wt%; or 5 wt% to 10 wt%. Within the above range, the flavor expression function according to the thermal decomposition of the flavoring agent may be obtained, and when applied to smoking articles, the effect of improving cigarette taste may be obtained.

According to one embodiment of the present disclosure, the composition is prepared in various phases, and may be, for example, a solid phase (e.g., powder, crystal, flake, and pulverized material), slurry, paste, gel, liquid phase, emulsion, or aerosol. For example, the composition may be molded, mixed into a desired product, or applied in a manner known in the art of the present disclosure such as printing, dipping, spraying, and/or coating, but is not specifically mentioned in this document.

The present disclosure relates to a food containing the flavoring agent according to the present disclosure. According to one embodiment of the present disclosure, the food may contain at least one of the above-mentioned flavoring compounds represented by Formula 1 according to the present disclosure. During heating and/or combustion of the food, flavors by thermal decomposition of the flavoring agent may be provided.

According to one embodiment of the present disclosure, the food may be prepared as a food raw material and the flavoring agent or a composition including the flavoring agent, and the composition may further include a food additive according to the desired food. For example, the composition may include solvents (e.g., water, alcohols, and liquid extracts), binders, diluents (e.g., oils), decomposing agents, lubricants, colorants, preservatives, antioxidants, emulsifiers, stabilizers, flavor enhancers, and sweeteners, but is not limited thereto. For example, the flavoring agent itself may be incorporated into the food, or the flavoring agent may be mixed, dipped, sprayed, and/or coated using a composition including the flavoring agent.

According to one embodiment of the present disclosure, the food may be semi-cooked or cooked by adding or heating the flavoring agent. When the flavoring agent is added, the food may express a flavoring agent function through additional heating. In addition, a half-cooked or cooked food is heated together with the flavoring agent to express its function, and the flavoring agent is added to the half-cooked or cooked food to enable its function to be expressed through additional heating.

According to one embodiment of the present disclosure, the flavoring agent may be contained in the food in an amount of 0.0001 wt% or more to 99 wt%; 0.01 wt% or more; 0.1 wt% or more; 1 wt% to 50 wt%; 1 wt% to 30 wt%; or 1 wt% to 20 wt%. Within the above range, it may be possible to provide the function of expressing the flavor of the flavoring agent and to maintain the inherent characteristics of the food raw material.

According to one embodiment of the present disclosure, the "food" may be food ingredients, sauces, additives, seasonings, food and beverages, favorite foods, processed foods, frozen foods, refrigerated foods, stored foods, pickled foods, functional foods, or fermented foods. In addition, the "food" may be uncooked and added state (e.g., surface coated state, filled state, seasoned state, pickled state, dried state, or mixed state), and semi-cooked or cooked foods (e.g., foods completed by applying heat such as baking, steaming, roasting, frying, boiling, or heating). For example, the "food" may be cereal products, rice products, tapioca products, sago products, bakery products, rice cake products, biscuit products, pastry products, candy products, dessert products, gum, chewing gum, chocolate, ice cream, honey products, molasses products, yeast products, baking powders, salt and seasoning products, condiments, sweeteners, savory products, mustard products, vinegar products, sauces (condiments), cooked fruit and vegetable products, and meat products, jellies, jams, fruits sauces, egg products, milk and dairy products, cheese products, butter and butter substitutes, milk substitutes, soy products, edible oils and fat products, beverages, alcoholic beverages, beer, carbonated beverages, carbonated water and other non-alcoholic beverages, fruit drinks, fruit juice, coffee, artificial coffee, tea, cocoa, chocolate, candy, extract foods, plant extracts, meat extracts, gelatin, medicines, elixirs, syrups, or other preparations for beverage preparation, but is not limited thereto.

The present disclosure relates to a smoking article including a flavoring agent according to the present disclosure. According to one embodiment of the present disclosure, the smoking article may include at least one of the above-mentioned flavoring compounds represented by Formula 1 according to the present disclosure. Flavor may be provided by thermal decomposition of the flavoring agent upon heating and/or combustion of the smoking article. That is, flavors may be developed in the mainstream smoke and/or sidestream smoke during heating and/or combustion of the smoking article.

According to one embodiment of the present disclosure, the "smoking article" may mean any smokable product or any product that may provide a smoking experience regardless of whether or not it is based on tobacco, tobacco derivatives, expanded tobacco, reconstituted tobacco, or tobacco substitutes. For example, the smoking article may mean a smokable article capable of generating an aerosol, such as a cigarette, cigar, cigarillo, and electronic cigarette. The smoking article may include an aerosol-generating material or an aerosol-forming substrate. In addition, the smoking articles may include solid materials based on tobacco raw materials, such as sheet-shaped tobacco, cut tobacco, and reconstituted tobacco.

According to one embodiment of the present disclosure, the flavoring agent may be contained in an amount of 0.0001 parts by weight or more; 0.001 parts by weight or more; 0.1 parts by weight or more; 1 part by weight or more; or 1 part by weight to 20 parts by weight based on 100 parts by weight of the smoking medium in the smoking article.

According to one embodiment of the present disclosure, the flavoring agent may develop flavor ingredients, for example, lactones in an amount of 0.00001 part by weight or more; 0.0001 parts by weight or more; 0.001 parts by weight or more; 0.1 parts by weight or more; 1 part by weight or more; or 1 part by weight to 20 parts by weight based on 100 parts by weight of the smoking medium in the smoking article during smoking.

According to one embodiment of the present disclosure, the smoking article may be a cigarette type tobacco, liquid type tobacco, or hybrid type tobacco, and may be a combustion type cigarette or heating type tobacco. Alternatively, the smoking article may be an electronic cigarette (e.g., electronically heated cigarette).

According to one embodiment of the present disclosure, the flavoring agent is applied to the cigarette paper of a cigarette so that flavor ingredients (e.g., lactones and/or fragrance ingredients) are developed by heat during heating and/or combustion of tobacco, particularly during smoke smoldering, and thus the effect of relieving the acrid smell of sidestream smoke may be enhanced.

According to one embodiment of the present disclosure, when applied to a medium of a heating-type tobacco stick, the persistence of the flavor ingredients may be imparted. That is, in the heating-type tobacco, the flavor ingredients contained in the medium are exhausted in the initial puff by static heating, but since the flavor ingredients are developed only when the flavoring agent is decomposed by heat, the flavor ingredients are produced also in the last puff even if the puff continues, and thus the cigarette taste may be constantly maintained.

According to one embodiment of the present disclosure, the smoking article may include or be prepared the flavoring agent or the flavoring agent composition. For example, the smoking article may correspond to a component and/or part. The smoking article may preferably be a component and/or part of the region to be heated. For example, the smoking article may be smoking media (e.g., liquid phases, gels, solid phases, slurries, and pastes), paper tubes, tubes, filters (e.g., tube filters, fabric filters, woven fabric filters, paper filters, and capsule filters), wrapping paper, cigarette paper, tip paper, wrapper, and cartridge (e.g., heating cartridge). The smoking article includes components known in the art of the present disclosure, and unless it departs from the object of the present disclosure, it is not specifically mentioned in this document.

According to one embodiment of the present disclosure, when the smoking article is manufactured, the flavoring agent may be applied by mixing the flavoring agent itself with a substrate or base material, or by mixing, printing, dipping (or, impregnating), coating and/or spraying with the substrate or base material using a composition including the flavoring agent. According to one embodiment of the present disclosure, the smoking medium, e.g., a flavoring agent and tobacco raw materials (e.g., medium raw material, and tobacco leaves) may be contained, or additives may be further contained. In another example, the flavoring agent may be added as a flavoring agent when manufacturing components and/or parts of the smoking article, and may be mixed with a base material, a solvent, a flavoring material, and a smoking medium material that are applicable to the smoking article. Alternatively, the smoking medium may be a liquid phase, gel or solid phase.

Hereinafter, the present disclosure will be described in more detail by examples and comparative examples. However, the following examples are only for illustrating the present disclosure, and the content of the present disclosure is not limited to the following examples.

### Example 1

### 1. Synthesis of sodium(4-mentylcarbonyloxy)heptanoate (5a)

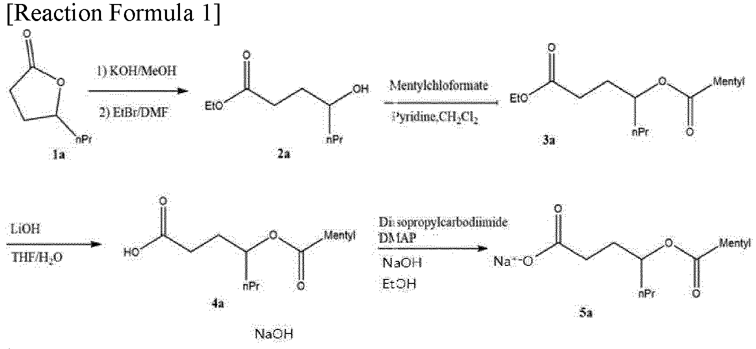

### (1-1) Synthesis of ethyl 4-hydroxyheptanoate (2a)

20 g (0.15 mol) of γ-heptalactone was dissolved in 100 mL of methanol, and while stirring the dissolved solution, 11.17 g (0.16 mol, 1.05 eq.) of KOH was slowly put thereinto and reacted at room temperature for 12 hours. After concentrating the reaction solution under reduced pressure, 80 mL of DMF was put thereinto, and while stirring the mixed solution, 17 g (0.15 mol, 1 eq.) of bromoethane was put thereinto, and reacted for 12 hours. 100 mL of water was put into the reaction solution, extracted with ethyl acetate, and then washed with water and brine. The organic layer was dried over MgSO₄ and then concentrated under reduced pressure to obtain 18.1 g (66.7%, 2 steps) of the target product 2a.
¹H NMR(CDCl₃, 400.13 MHz); δ 8.01(s, 1H, -OH), 4.12(q, 2H, J = 8 Hz, COO-CH₂-), 3.63(m, 1H, CH-O), 2.42(m, 2H, CO-CH₂), 1.81 to 0.92(m, 12H, alkyl)

### (1-2) Synthesis of ethyl 4-(mentylcarbonyloxy)heptanoate (3a)

18 g (0.1 mol) of ethyl 4-hydroxyheptanoate (2a) was dissolved in 120 mL of THF, 16 g (0.2 mol, 2 eq.) of pyridine was put into the dissolved solution and cooled with ice water, and while stirring the cooled mixed solution, 23 g (0.1 mol, 1 eq.) of methyl chloroformate and 20 mL of THF were slowly dropped thereinto. After 1 hour, the reaction solution was raised to room temperature and reacted overnight, and then water was put thereinto and extracted with ethyl acetate. The organic layer was washed with dilute hydrochloric acid, a saturated sodium bicarbonate solution, and brine, respectively, dried over MgSO₄, and concentrated under reduced pressure to obtain 30 g (yield of 81%) of the target product 3a as a yellow liquid.
¹H NMR(CDCl₃, 400.13 MHz); δ 4.74(7tet, 1H, J = 4 Hz, -COOCH-), 4.51(td, 1H, J = 9, 4 Hz, COO-CH-), 4.12(q, 2H, J = 8 Hz, COO-CH₂ -), 2.36(m, 2H, CO-CH₂-), 1.93 to 0.79(m, 30H, alkyl)

### (1-3) Synthesis of 4-(mentylcarbonyloxy)heptanoic acid (4a)

25 g (68.5 mmol) of ethyl 4-(mentylcarbonyloxy)heptanoate (3a) was dissolved in 100 mL of THF and 30 mL of distilled water, and 4.2 g (102.4 mmol, 1.5 eq.) of lithium hydroxide monohydrate was put into the dissolved solution and reacted at room temperature for 12 hours. 50 mL of distilled water was added to the reaction solution and extracted with ether. The aqueous layer was adjusted to pH 3 by putting concentrated hydrochloric acid thereinto and then extracted with ethyl acetate. After the organic layer was washed with brine, the washed organic layer was dried over MgSO₄ and concentrated under reduced pressure to obtain 21.8 g (yield of 81%) of the target product 4a as a yellow liquid.
¹H NMR(CDCl₃, 400.13 MHz); δ 4.76(m, 1H, -COOCH-), 4.52(td, 1H, J = 9, 4 Hz, COO-CH-), 4.11(q, 2H, J = 8 Hz, COO-CH₂-), 2.42( m, 2H, CO-CH₂-), 1.99 to 0.82(m, 27H, alkyl)

### (1-4) Synthesis of sodium(4-mentylcarbonyloxy)heptanoate (5a)

2.5 g (7.5 mmol) of 4-(mentylcarbonyloxy)heptanoic acid (4a) was dissolved in 20 mL of 95% ethanol, and 0.29 g (0.95 eq.) of 98% NaOH was put into the dissolved solution, and stirred at room temperature for 2 hours. Water and ethanol were blown away using an azeotropic phenomenon, toluene was added to remove water, and then hexane and ethyl acetate were put thereinto and filtered to obtain a white solid.

### 2. Synthesis of sodium 4-(mentylcarbonyloxy)nonanoate (5b)

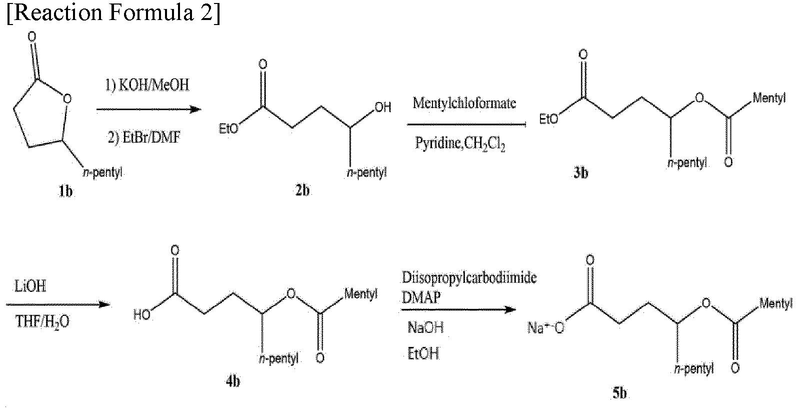

### (2-1) Synthesis of ethyl 4-hydroxynonanoate (2b)

20 g (0.13 mol) of γ-nonalactone was dissolved in 100 mL of methanol, and while stirring the dissolved solution, 9.18 g (0.14 mol, 1.05 eq.) of KOH was slowly put thereinto and reacted at room temperature for 12 hours. After concentrating the reaction solution under reduced pressure, 80 mL of DMF was put thereinto, and while stirring the mixed solution, 14 g (0.13 mol, 1 eq.) of bromoethane was put thereinto, and reacted for 12 hours. 100 mL of water was put into the reaction solution, extracted with ethyl acetate, and then washed with water and brine. The organic layer was dried over MgSO₄ and then concentrated under reduced pressure to obtain 24 g (93%, 2 steps) of the target product 2b.

### (2-2) Synthesis of ethyl 4-(mentylcarbonyloxy)nonanoate (3b)

24 g (0.12 mol) of ethyl 4-hydroxynonanoate (2b) was dissolved in 120 mL of THF, 18 g (0.42 mol, 2 eq.) of pyridine was put into the dissolved solution and cooled with ice water, and while stirring the cooled mixed solution, 26 g (0.12 mol, 1 eq.) of methyl chloroformate and 30 mL of THF were slowly dropped thereinto. After 1 hour, the reaction solution was raised to room temperature and reacted overnight, and then water was put thereinto and extracted with ethyl acetate. The organic layer was washed with dilute hydrochloric acid, a saturated sodium bicarbonate solution, and brine, respectively, dried over MgSO₄, and concentrated under reduced pressure to obtain 34 g (yield of 74.5%) of the target product 3b as a yellow liquid.
¹H NMR(CDCl₃, 400.13 MHz); δ 4.74(7tet, 1H, J = 4 Hz, -COOCH-), 4.51(td, 1H, J = 9, 4 Hz, COO-CH-), 4.12(q, 2H, J = 8 Hz, COO-CH2 -), 2.36(m, 2H, CO-CH₂-), 1.93 to 0.79(m, 23H, alkyl)

### (2-3) Synthesis of 4-(mentylcarbonyloxy)nonanoic acid (4b)

11.5 g (29.9 mmol) of ethyl 4-(mentylcarbonyloxy)nonanoate (3b) was dissolved in 50 mL of THF and 20 mL of distilled water, and 2 g (48.7 mmol, 1.6 eq.) of lithium hydroxide monohydrate was put into the dissolved solution and reacted at room temperature for 12 hours. 50 mL of distilled water was added to the reaction solution and extracted with ether. The aqueous layer was adjusted to pH 3 by putting concentrated hydrochloric acid thereinto and then extracted with ethyl acetate. After the organic layer was washed with brine, the washed organic layer was dried over MgSO₄ and concentrated under reduced pressure to obtain 8.6 g (yield of 80%) of the target product 4b as a yellow liquid.
¹H NMR(CDCl₃, 400.13 MHz); δ 4.75(m, 1H, -COOCH-), 4.49(m, 1H, COO-CH-), 2.04(m, 2H, CO-CH₂-), 1.93 to 0.79 (m, 31H, alkyl)

### (2-4) Synthesis of sodium 4-(mentylcarbonyloxy)nonanoate (5b)

2.5 g (7.5 mmol) of 4-(mentylcarbonyloxy)nonanoic acid (4b) was dissolved in 20 mL of 95% ethanol, and 0.29 g (0.95 eq.) of 98% NaOH was put into the dissolved solution, and stirred at room temperature for 2 hours. Water and ethanol were blown away using an azeotropic phenomenon, toluene was added to remove water, and then hexane and ethyl acetate were put thereinto and filtered to obtain a white solid.

### 3. Sodium 5-(mentylcarbonyloxy)decanoate (5c)

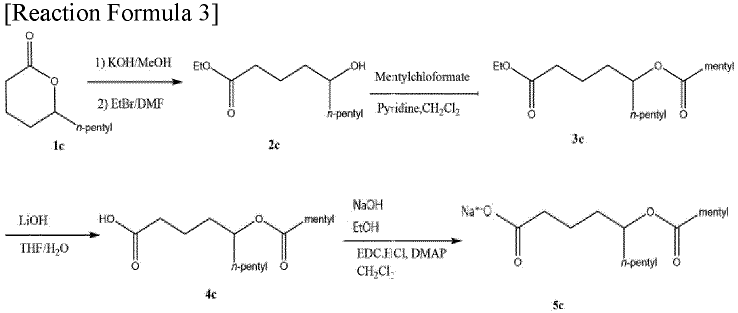

### (3-1) Synthesis of ethyl 5-hydroxydecanoate (2c)

10 g (58.7 mmol) of δ-decalactone was dissolved in 50 mL of methanol, and while stirring the dissolved solution, 4.2 g (64.7 mmol, 1.05 eq.) of KOH was slowly put thereinto and reacted at room temperature for 12 hours. After concentrating the reaction solution under reduced pressure, 40 mL of DMF was put thereinto, and while stirring the mixed solution, 6.4 g (58.7 mmol, 1 eq) of bromoethane was put thereinto, and reacted for 12 hours.
100 mL of water was put into the reaction solution, extracted with ethyl acetate, and then washed with water and brine. The organic layer was dried over MgSO₄ and then concentrated under reduced pressure to obtain 7.6 g (60%, 2 steps) of the target product 2c.

### (3-2) Synthesis of ethyl 5-(mentylcarbonyloxy)decanoate (3c)

7.5 g (34.6 mmol) of ethyl 5-hydroxydecanoate (2c) was dissolved in 50 mL of THF, 5.3 g (69.2 mmol, 2 eq.) of pyridine was put into the dissolved solution and cooled with ice water, and while stirring the cooled mixed solution, 8.3 g (37.9 mmol, 1.1 eq.) of methyl chloroformate and 20 mL of THF were slowly dropped thereinto. After 1 hour, the reaction solution was raised to room temperature and reacted overnight, and then water was put thereinto and extracted with ethyl acetate. The organic layer was washed with dilute hydrochloric acid, a saturated sodium bicarbonate solution, and brine, respectively, dried over MgSO₄, and concentrated under reduced pressure. The mixture was subjected to silica gel column chromatography using a mixed solvent of n-hexane and ethyl acetate (7:1) to obtain 4.5 g (yield of 32.6%) of the target product 3c.
¹H NMR(CDCl₃, 400.13 MHz); δ 4.72(m, 1H, -COOCH-), 4.52(m, 1H, COO-CH-), 4.12(q, 2H, J = 8 Hz, COO-CH₂-), 2.31(t, 2H, J = 8 Hz, CO-CH₂-), 2.08 to 0.86(m, 27H, alkyl), 0.79(d, 6H, J = 8 Hz, -CH₃).

### (3-3) Synthesis of 5-(mentylcarbonyloxy)decanoic acid (4c)

2.7 g (6.8 mmol) of ethyl 4-(methylcarbonyloxy)nonanoate (3c) was dissolved in 20 mL of THF and 10 mL of distilled water, and 0.42 g (10.2 mmol, 1.5 eq.) of lithium hydroxide monohydrate was put into the dissolved solution and reacted at room temperature for 12 hours. 10 mL of distilled water was added to the reaction solution and extracted with ether. The aqueous layer was adjusted to pH 3 by putting concentrated hydrochloric acid thereinto and then extracted with ethyl acetate. After the organic layer was washed with brine, the washed organic layer was dried over MgSO₄ and concentrated under reduced pressure to obtain 2.1 g (yield of 78%) of the target product 4c as a yellow liquid.
¹H NMR(CDCl₃, 400.13 MHz); δ 4.72(m, 1H, -COOCH-), 4.51(td, 1H, J = 8, 4 Hz, COO-CH-), 4.11(q, 2H, J = 8 Hz, COO-CH₂-), 2.38( m, 2H, CO-CH₂-), 2.06 to 0.78(m, 33H, alkyl)

### (3-4) Synthesis of sodium 5-(mentylcarbonyloxy)decanoate (5c)

7.5 mmol of 5-(mentylcarbonyloxy)decanoic acid (4c) was dissolved in 20 mL of 95% ethanol, and 0.29 g (0.95 eq.) of 98% NaOH was put into the dissolved solution, and stirred at room temperature for 2 hours. Water and ethanol were blown away using an azeotropic phenomenon, toluene was added to remove water, and then hexane and ethyl acetate were put thereinto and filtered to obtain a white solid.

### 4. Synthesis of sodium(4-mentylcarbonyloxy)undecanoate (5d)

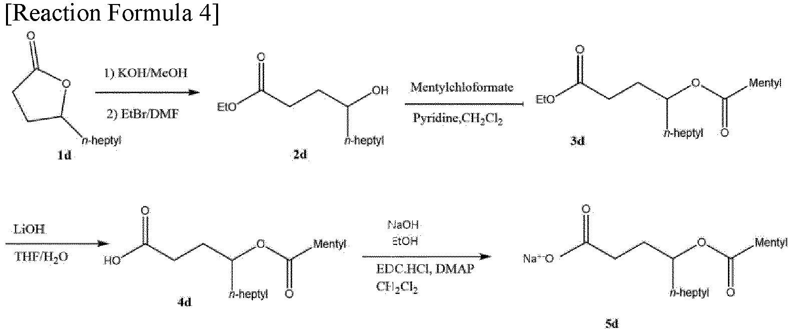

### (4-1) Synthesis of ethyl 4-hydroxyundecanoate (2d)

10 g (54.2 mmol) of γ-undecalactone was dissolved in 50 mL of methanol, and while stirring the dissolved solution, 3.9 g (56.9 mmol, 1.05 eq.) of KOH was slowly put thereinto and reacted at room temperature for 12 hours. After concentrating the reaction solution under reduced pressure, 50 mL of DMF was put thereinto, and while stirring the mixed solution, 5.9 g (54.2 mmol, 1 eq.) of bromoethane was put thereinto, and reacted for 12 hours. 80 mL of water was put into the reaction solution, extracted with ethyl acetate, and then washed with water and brine. The organic layer was dried over MgSO₄ and then concentrated under reduced pressure to obtain 10.7 g (85.6%, 2 steps) of the target product 2d.
¹H NMR(CDCl₃, 400.13 MHz); δ 4.12(q, 2H, J = 8 Hz, COO-CH₂-), 3.59(m, 1H, CH-O), 2.43(m, 2H, CO-CH₂), 1.81 to 0.92(m, 20H, alkyl)

### (4-2) Synthesis of ethyl 4-(mentylcarbonyloxy)undecanoate (3d)

11 g (47.7 mmol) of ethyl 4-hydroxyundecanoate (2d) was dissolved in 60 mL of THF, 6.8 g (95.5 mmol, 2 eq.) of pyridine was put into the dissolved solution and cooled with ice water, and while stirring the cooled mixed solution, 10.5 g (47.7 mmol, 1 eq.) of mentyl chloroformate and 20 mL of THF were slowly dropped thereinto. After 1 hour, the reaction solution was raised to room temperature and reacted overnight, and then water was put thereinto and extracted with ethyl acetate. The organic layer was washed with dilute hydrochloric acid, a saturated sodium bicarbonate solution, and brine, respectively, dried over MgSO₄, and concentrated under reduced pressure to obtain 8.3 g (yield of 42.1%) of the target product 3d as a yellow liquid.
¹H NMR(CDCl₃, 400.13 MHz); δ 4.74(7tet, 1H, J = 4 Hz, -COOCH-), 4.51(td, 1H, J = 9, 4 Hz, COO-CH-), 4.12(q, 2H, J = 8 Hz, COO-CH₂-), 2.36(m, 2H, CO-CH₂-), 1.93 to 0.79 (m, 23H, alkyl)

### (4-3) Synthesis of 4-(mentylcarbonyloxy)undecanoic acid (4d)

8.3 g (19.4 mmol) of ethyl 4-(mentylcarbonyloxy)undecanoate (3d) was dissolved in 30 mL of THF and 20 mL of distilled water, and 1.2 g (29.1 mmol, 1.5 eq.) of lithium hydroxide monohydrate was put into the dissolved solution and reacted at room temperature for 12 hours. 20 mL of distilled water was added to the reaction solution and extracted with ether. The aqueous layer was adjusted to pH 3 by putting concentrated hydrochloric acid thereinto and then extracted with ethyl acetate. After the organic layer was washed with brine, the washed organic layer was dried over MgSO₄ and concentrated under reduced pressure. The mixture was subjected to silica gel column chromatography using a mixed solvent of hexane (n-hexane) and ethyl acetate (8:1) to obtain 6.8 g (yield of 91.8%) of the target product 4d.
¹H NMR(CDCl₃, 400.13 MHz); δ 4.75(m, 1H, -COOCH-), 4.51(m, 1H, COO-CH-), 2.43(m, 2H, CO-CH₂-), 2.17 to 0.78(m, 35H, alkyl)

### (4-4) Synthesis of sodium(4-mentylcarbonyloxy)undecanoate (5d)

2.5 g (7.5 mmol) of 4-(mentylcarbonyloxy)undecanoic acid (4d) was dissolved in 20 mL of 95% ethanol, and 0.29 g (0.95 eq.) of 98% NaOH was put into the dissolved solution, and stirred at room temperature for 2 hours. Water and ethanol were blown away using an azeotropic phenomenon, toluene was added to remove water, and then hexane (n-hexane) and ethyl acetate were put thereinto and filtered to obtain a white solid.

### 5. Synthesis of sodium 4-(benzyloxycarbonyloxy)undecanoate (5e)

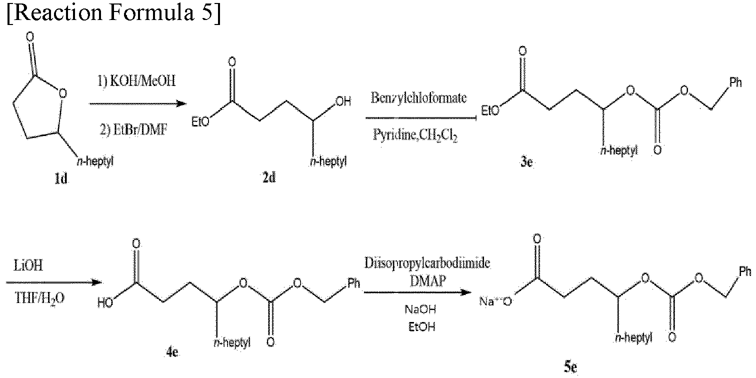

### (5-1) Synthesis of ethyl 4-hydroxyundecanoate (2d)

10 g (54.2 mmol) of γ-undecalactone was dissolved in 50 mL of methanol, and while stirring the dissolved solution, 3.9 g (56.9 mmol, 1.05 eq.) of KOH was slowly put thereinto and reacted at room temperature for 12 hours. After concentrating the reaction solution under reduced pressure, 50 mL of DMF was put thereinto, and while stirring the mixed solution, 5.9 g (54.2 mmol, 1 eq.) of bromoethane was put thereinto, and reacted for 12 hours.

80 mL of water was put into the reaction solution, extracted with ethyl acetate, and then washed with water and brine. The organic layer was dried over MgSO₄ and then concentrated under reduced pressure to obtain 10.7 g (85.6%, 2 steps) of the target product 2d.

¹H NMR(CDCl₃, 400.13 MHz); δ 4.12(q, 2H, J = 8 Hz, COO-CH₂-), 3.59(m, 1H, CH-O), 2.43(m, 2H, CO-CH₂), 1.81 to 0.92(m, 20H, alkyl)

### (5-2) Synthesis of ethyl 4-(benzyloxycarbonyloxy)undecanoate (3e)

8.3 g (36 mmol) of ethyl 4-hydroxyundecanoate (2d) was dissolved in 50 mL of THF, 5.5 g (72.3 mmol, 2 eq.) of pyridine was put into the dissolved solution and cooled with ice water, and while stirring the cooled mixed solution, 6.1 g (35.3 mmol, 1 eq.) of benzyl chloroformate and 20 mL of THF were slowly dropped thereinto. After 1 hour, the reaction solution was raised to room temperature and reacted overnight, and then water was put thereinto and extracted with ethyl acetate. The organic layer was washed with dilute hydrochloric acid, a saturated sodium bicarbonate solution, and brine, respectively, dried over MgSO₄, and concentrated under reduced pressure to obtain 9.9 g (yield of 75.6%) of the target product 3e as a yellow liquid. ¹H NMR(CDCl₃, 400.13 MHz); δ 7.37 to 7.34(m, 5H, ph), 5.14(m, 2H, O-CH2-Ph), 4.12(brs, 1H, O-CH-), 2.42(m, 2H, CO-CH₂-), 1.90 to 0.79(m, 21H, alkyl) (FIG. 24).

### (5-3) Synthesis of 4-(benzyloxycarbonyloxy)undecanoic acid (4e)

10 g (27.5 mmol) of ethyl 4-(benzyloxycarbonyloxy)undecanoate (3e) was dissolved in 30 mL of THF and 20 mL of distilled water, and 1.7 g (41.4 mmol, 1.5 eq.) of lithium hydroxide monohydrate was put into the dissolved solution and reacted at room temperature for 12 hours. 20 mL of distilled water was added to the reaction solution and extracted with ether. The aqueous layer was adjusted to pH 3 by putting concentrated hydrochloric acid thereinto and then extracted with ethyl acetate. After the organic layer was washed with brine, the washed organic layer was dried over MgSO₄ and concentrated under reduced pressure to obtain 8.2 g (yield of 89%) of the target product 4e.
¹H NMR (CDCl₃, 400.13 MHz); δ 7.37 to 7.35(m, 5H, ph), 5.14(m, 2H, O-CH₂-Ph), 4.48(m, 1H, O-CH-), 2.47(m, 2H, CO-CH₂-), 1.90 to 0.79(m, 21H, alkyl)

### (5-4) Synthesis of sodium 4-(benzyloxycarbonyloxy)undecanoate (5e)

2.5 g (7.5 mmol) of 4-(benzyloxycarbonyloxy)undecanoic acid (4e) was dissolved in 20 mL of 95% ethanol, and 0.29 g (0.95 eq.) of 98% NaOH was put into the dissolved solution, and stirred at room temperature for 2 hours. Water and ethanol were blown away using an azeotropic phenomenon, toluene was added to remove water, and then hexane (n-hexane) and ethyl acetate were put thereinto and filtered to obtain a white solid.

### Experimental Example

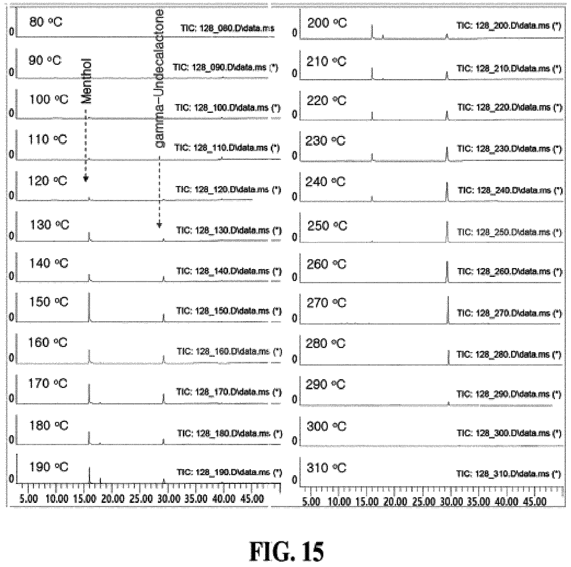

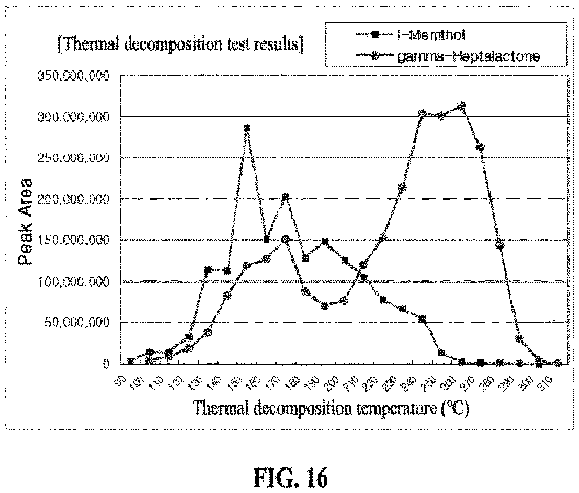

A pyrolysis test was conducted to confirm the pyrolytic behavior of the 5d Compound (2B) when exposed to heat, which was observed by a commonly-known pyrolysis-gas chromatography/mass spectrometry [Py-GC/ MS]. The pyrolyzer was performed in a system in which the 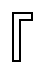 Double-Shot Pyrolyzer 2020iD 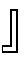 (Frontier Lab, Japan) was connected to the GC/MS (Agilent 6890 GC, USA/Aginelt 7890 MSD, USA) equipment. After diluting 2B to 2.5% concentration in an ethyl alcohol solution, 10 ul was loaded into a pyrolyzer sample cup, and then thermally decomposed. The temperature experienced by the sample was controlled by specifying the temperature of the furnace of the Double-Shot Pyrolyzer for the thermal decomposition temperature. The initial thermal decomposition temperature was set at 80°C for 30 seconds so that the Target Compound (2B) in the sample cup was allowed to undergo thermal decomposition by exposing the sample cup having a sample placed therein to the furnace. The components produced by heat or volatilized by heat were directly injected into the injector of GC/MS and separated. The sample cup was removed from the furnace during GC/MS analysis after thermal decomposition so that it was not affected by the thermal decomposition temperature, and after the GC/MS analysis by the first thermal decomposition was completed, the sample cup used for the first time was subjected to thermal decomposition again without injecting a new compound thereinto. At this time, the sample cup was subjected to thermal decomposition for 30 seconds at a thermal decomposition temperature of 90°C, which is 10°C higher. Also, after the thermal decomposition was completed, the sample cup was removed from the furnace so that it was not affected by the thermal decomposition temperature. In this manner, when the first sample was loaded into the sample cup and then thermally decomposed, the thermal decomposition experiment was performed while raising the temperature from 80°C, 90°C, and 100°C to 320°C in the end. As a result, it was possible to consider the thermal decomposition characteristics of compounds experienced as the thermal decomposition temperature increased by dividing them by temperature range. The results are shown in FIGS. 15 and 16.

### [Decomposition mechanism]

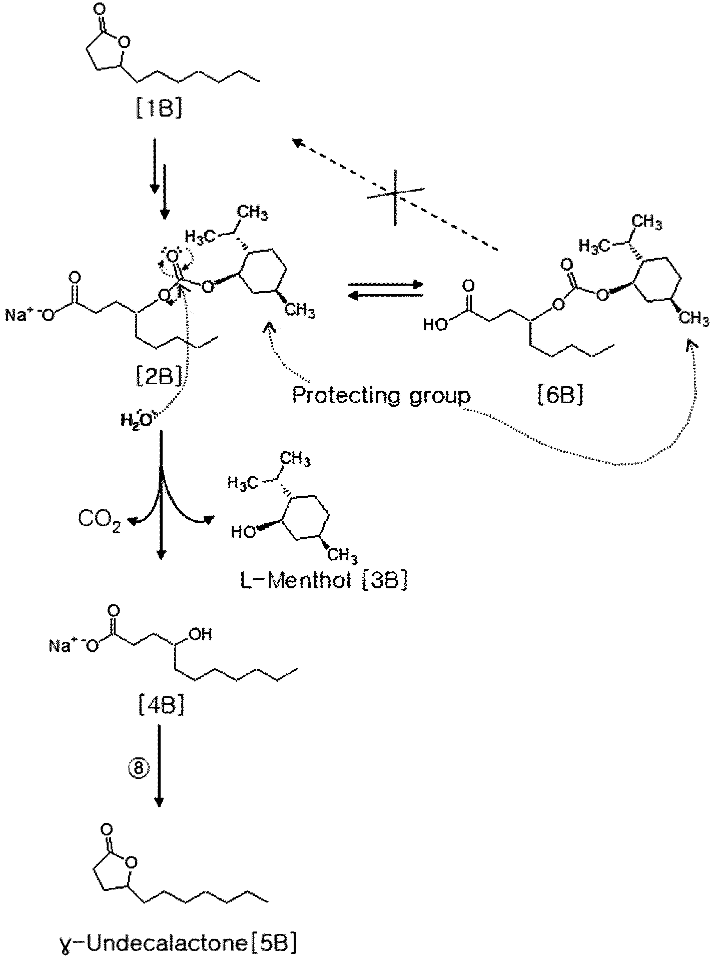

In FIGS. 15 and it may be confirmed that menthol and gamma-undecalactone decomposed at a temperature of approximately 120°C as a result of the thermal decomposition of Compound [2B].

That is, lactone [1B, gamma-undecalactone] the decomposition mechanism was 16, are test in ring-opened, and a hydroxyl group was covalently bonded with L-menthol through a carbonate linking group (carbonate linkage) to prepare Compound [2B]. After Compound [2B] is applied to the product matrix, Compound [4B] with exposed hydroxyl groups is formed as L-menthol ([3B]) and CO₂ are being generated by heat. Compound [4B] is also subjected to ring-closing (intramolecular esterification) by heat to produce gamma-undecalactone [5B]. In the [2B] state, the hydroxyl group is protected with a menthyl carbonate group so that the occurrence of ring-closing (intramolecular esterification) may be suppressed at room temperature. Looking at the thermal decomposition pattern of Compound [2B], menthol is thermally decomposed and expressed while it reaches a temperature from 120°C to 260°C, and gamma-lactone is first expressed while it reaches a temperature from 120°C to 200°C, and subsequently, secondary expression also becomes abundant while it reaches a temperature from 200°C to 300°C. Perhaps, even if menthol, which is used as a protecting group, is deprotected by heat and expressed, it seems to exist for a while as a compound state in the form of [4B], that is, as an intermediate state. Eventually, lactone is produced by intramolecular esterification, but this ring-closing may be retarded in the salt form state. In addition, as a result of the thermal decomposition experiment, as the temperature increased, menthol was thermally decomposed and expressed, and when it is in the [4B] state of the salt form, intramolecular esterification occurred at a little higher temperature to produce lactone [5B]. In other words, it could be found that remaining ring-closing occurs in a high temperature range with a time difference from the temperature range where menthol is thermally decomposed.

### Example 2

1 wt% of a target material of Preparation Example (synthesized sodium (4-methylcarbonyloxy)heptanoate (5a), 40 wt% of water, 10 wt% of milk, and 49 wt% of wheat flour were mixed and kneaded to prepare a dough, and the dough was baked by heating the dough in an electric oven at 200°C for 1 hour. After taking the baked dough out of the oven, it was confirmed that the flavor (e.g., lactone flavor and menthol flavor used in the synthesis of the target material) were expressed by sniffing it.

### Example 3

After mixing 0.01 to 5 wt% of a target material (synthesized sodium 5-(methylcarbonyloxy)decanoate (5c) of Preparation Example, 95 to 99 wt% of a base substrate (pulp), and the balance of other additives, the mixture was prepared into a sheet (2 mm thick) using roll-to-roll and dried at room temperature. The sheet was sniffed at room temperature, but there was no smell of the flavoring compounds used in the synthesis of the target material. Next, the sheet was applied as cigarette paper for cigarette tobacco to make a common cigarette, and the cigarette was smoked, and it was confirmed that flavors (e.g., lactone flavor and menthol flavor used in synthesizing the target material) were expressed during smoking.

### Example 4

After 0.003 to 0.02 wt% of a target material of Preparation Example (synthesized sodium (4-mentylcarbonyloxy)undecanoate) (5d), 90 to 99% by weight of tobacco powder with an average particle size of about 0.03 mm to about 0.12 mm, and the balance of other additives were mixed, a tobacco composition was prepared in the usual manner. After applying the tobacco composition as a smoking medium and wrapping it in cigarette paper, a filter and a wrapping paper were made up to prepare a usual cigarette tobacco. Cigarette tobacco was smoked, and it was confirmed that flavors were expressed during smoking in mainstream smoke and sidestream smoke.

## Claims

1. A flavoring agent, which is a compound represented by the following Formula 1: 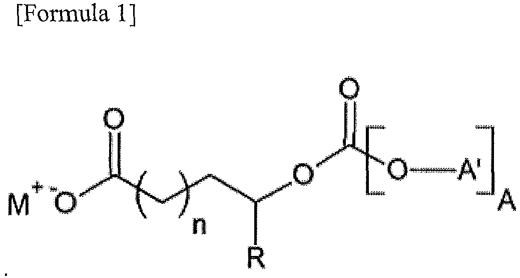 wherein in Formula 1, n is an integer of 1 or 2, M is selected from alkali metals and transition metals, R is a straight-chain or branched-chain alkyl group having 1 to 30 carbon atoms, and moiety A is a moiety derived from a flavoring compound having at least one of an aromatic ring, an aliphatic ring, and an aliphatic chain which have a hydroxyl group, wherein the hydroxyl group participates in a carbonate linking group ( 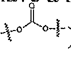 ), and A' corresponds to a flavoring compound except for the hydroxyl group.

2. The flavoring agent of claim 1, wherein the flavoring compound is selected from a cyclic monoterpene-based compound having a hydroxyl group, a monoterpene-based acyclic compound having a hydroxyl group, a C₆-C₁₀ aromatic compound having a hydroxyl group, and a C₅-C₆ non-aromatic ring having a hydroxyl group.

3. The flavoring agent of claim 1, wherein the flavoring compound is selected from the following formulas:

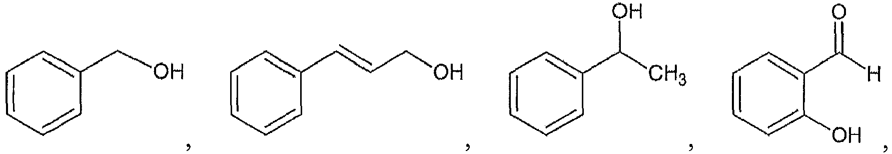

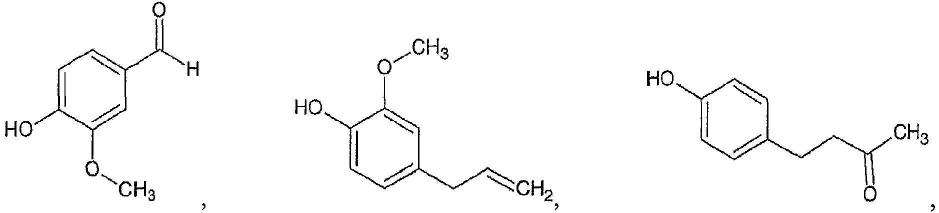

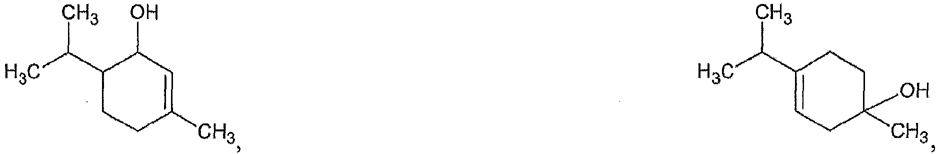

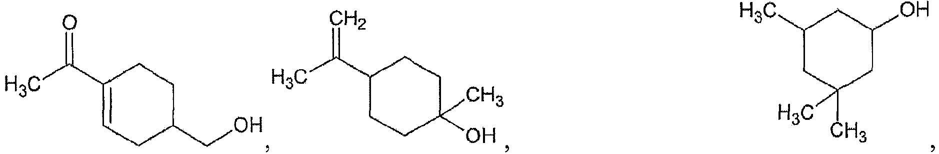

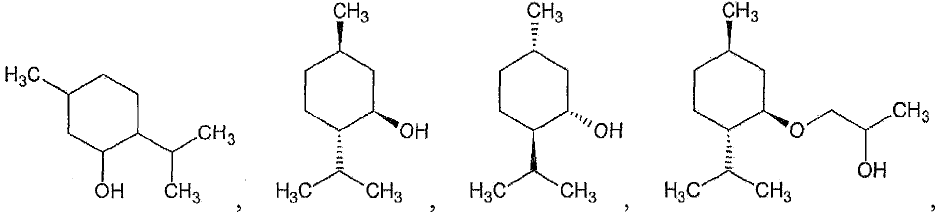

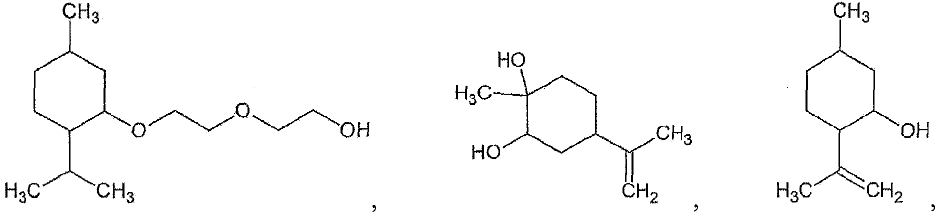

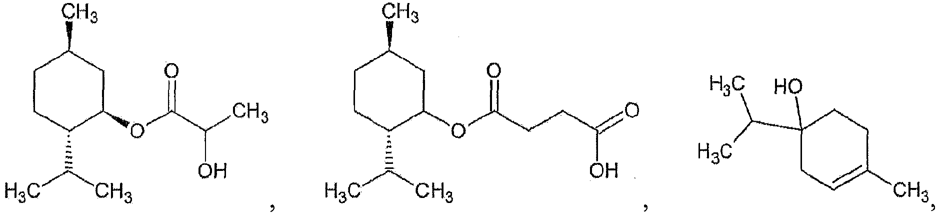

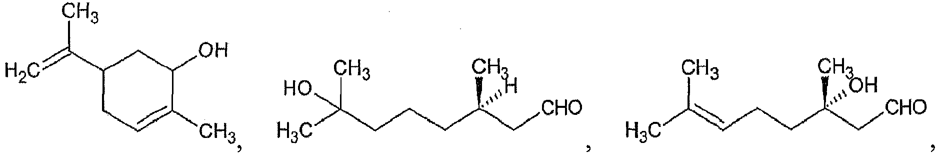

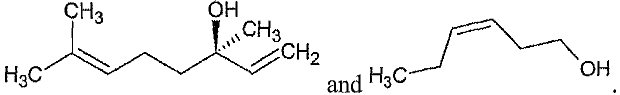

4. The flavoring agent of claim 1, wherein A' is selected from the following formulas, wherein * is the binding site of oxygen in the carbonate:

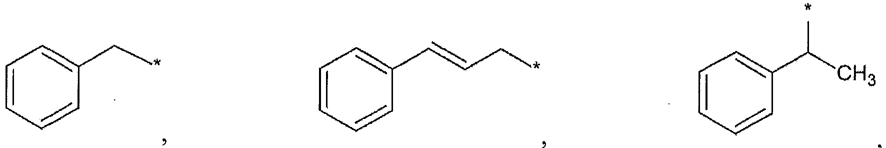

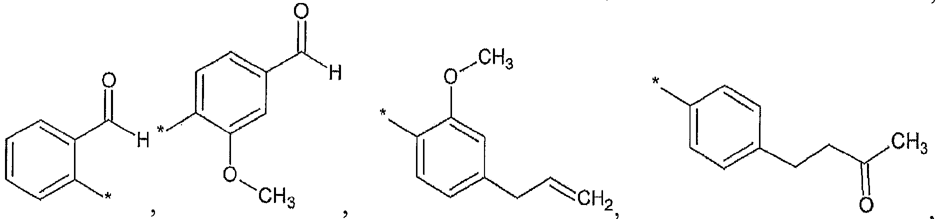

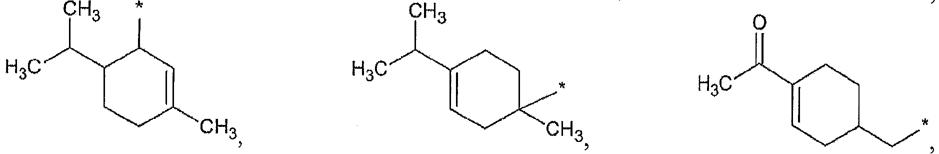

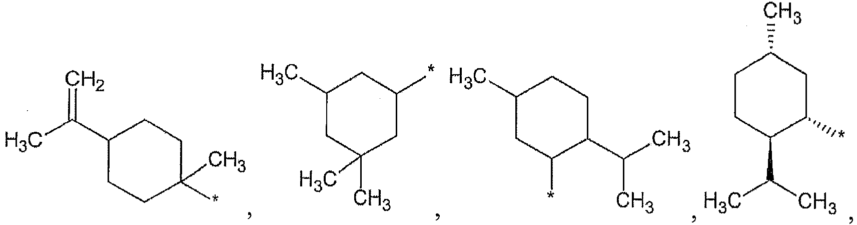

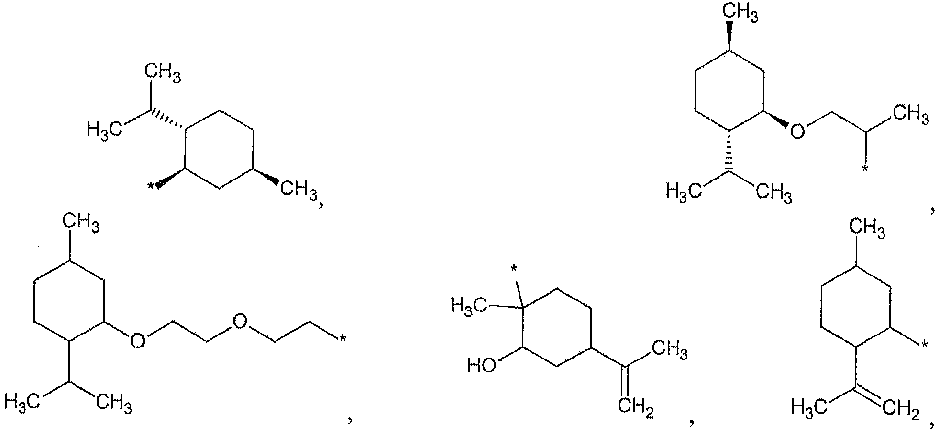

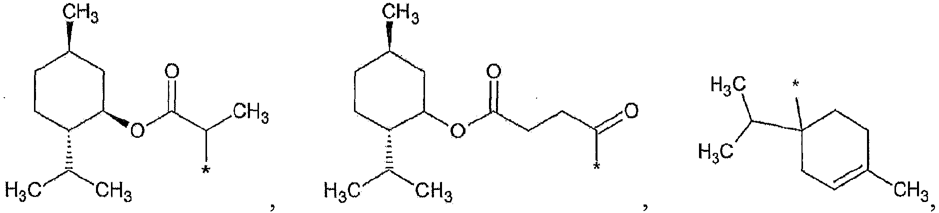

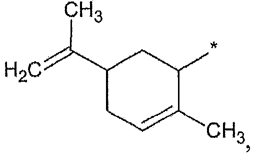

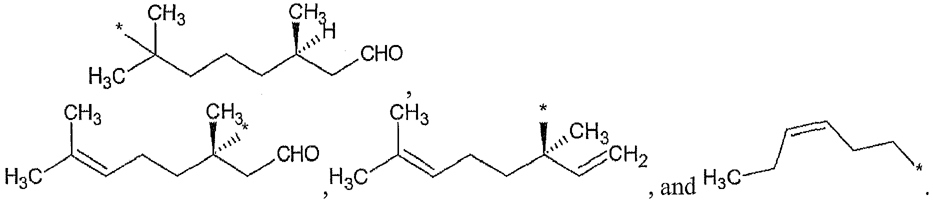

5. The flavoring agent of claim 1, wherein the transition metal is selected from Zr, Mg, Ca, Co, Rh, Ir, Nb, Pd, Pt, Fe, Ru, Os, Cr, Mo, W, Mn, Tc, Re, Cu, Ag, and Au, and the alkali metal is selected from Li, Na, K, Rb, and Cs.

6. The flavoring agent of claim 1, wherein the compound is selected from the following formulas 1-1 to 1-28: 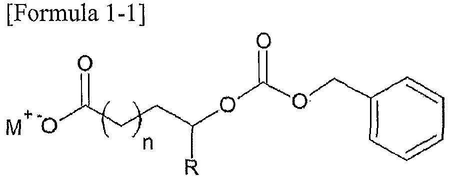 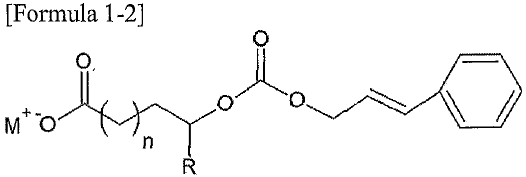 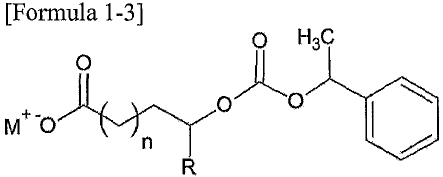 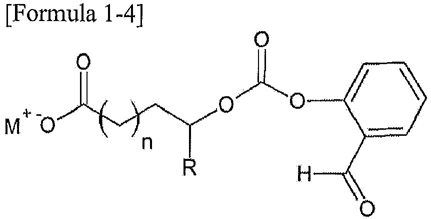 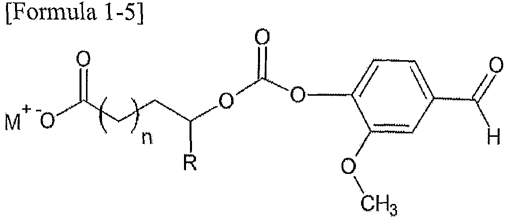 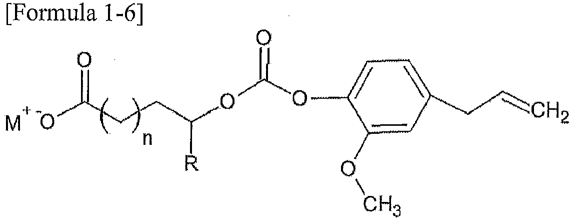 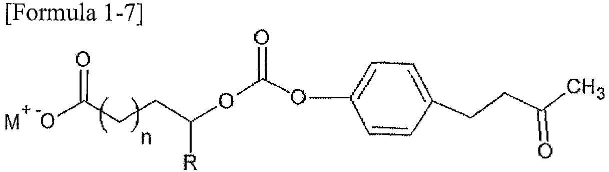 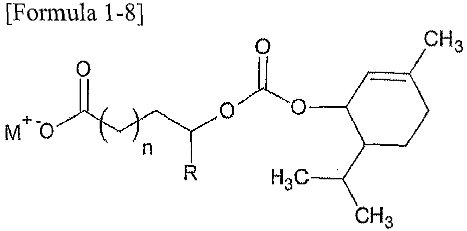 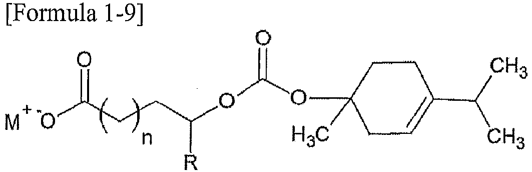 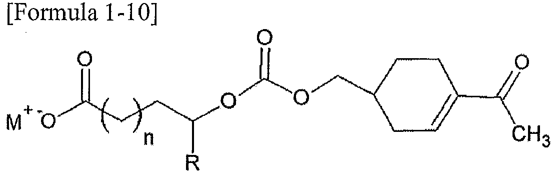 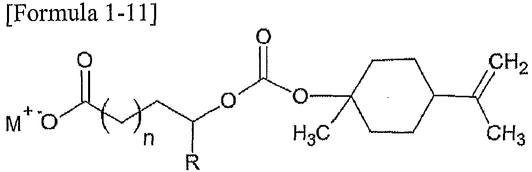 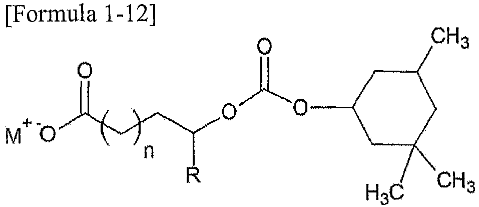 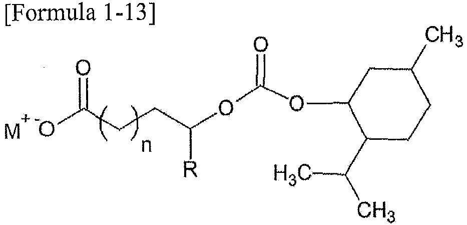 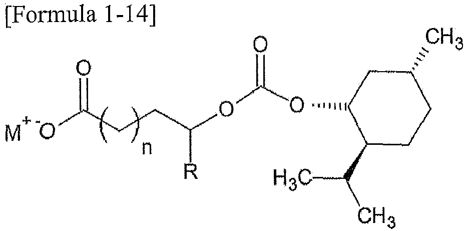 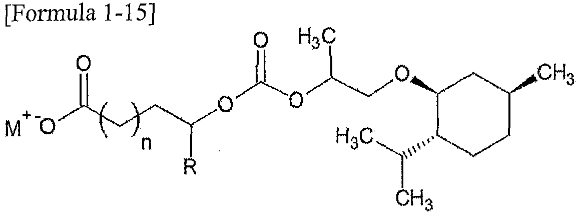 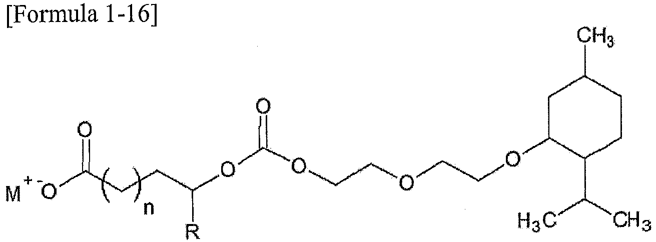 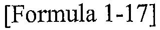 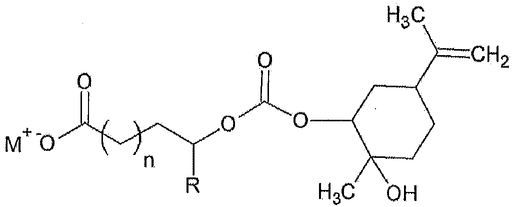 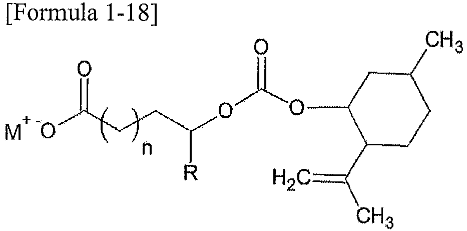 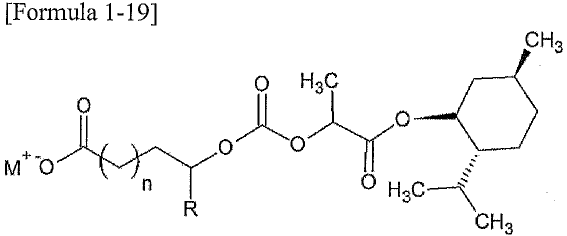 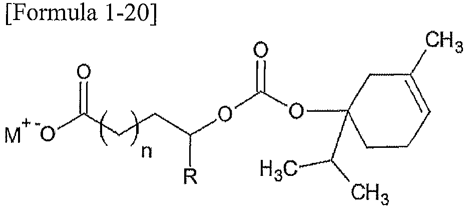 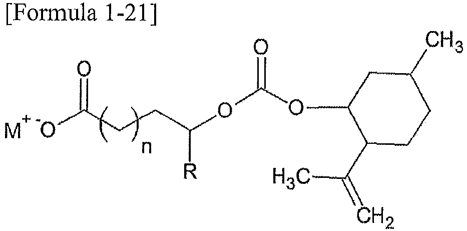 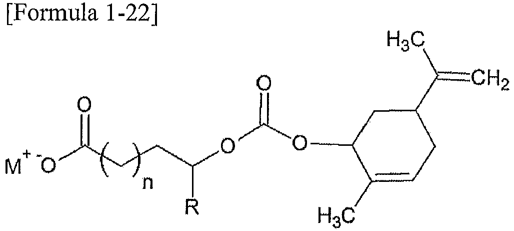 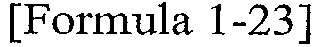 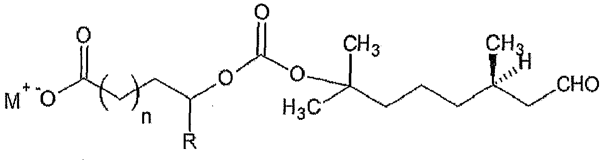 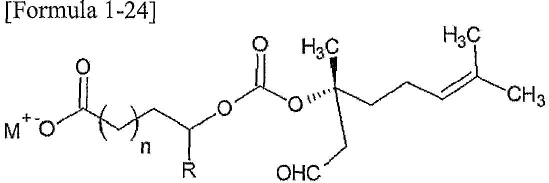 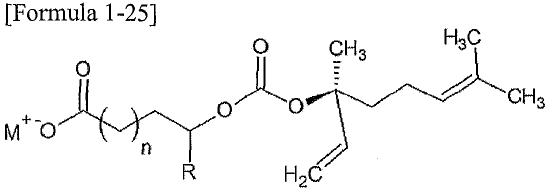 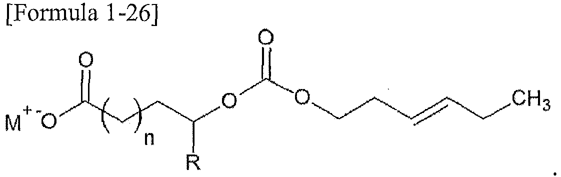 wherein M and R are as defined in Formula 1 above.

7. The flavoring agent of claim 1, wherein the flavoring agent is a flavoring agent compound that develops flavor upon thermal decomposition.

8. The flavoring agent of claim 1, wherein the flavoring agent is a flavoring agent that decomposes into the flavoring compound, a lactone compound, and carbon dioxide during thermal decomposition.

9. The flavoring agent of claim 1, wherein the compound is a compound that thermally decomposes at a temperature of 80°C or higher.

10. The flavoring agent of claim 8, wherein the lactone compound is gamma lactone of the following Formula 2 or delta lactone of the following Formula 3: 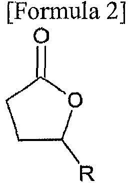 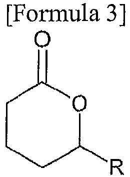 wherein R is a straight-chain or branched-chain alkyl group having 1 to 30 carbon atoms.

11. The flavoring agent of claim 8, wherein the lactone compound is selected from the following formulas: 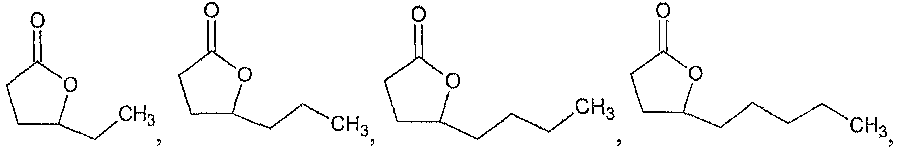 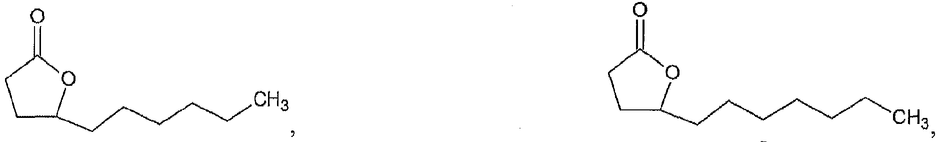 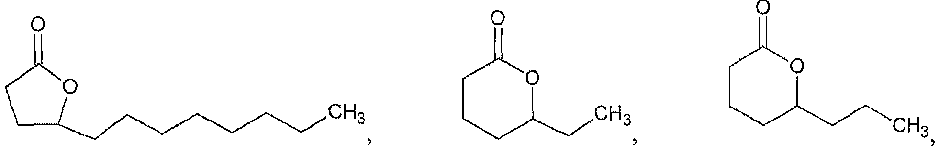 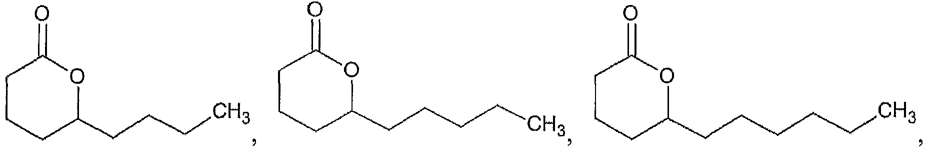 and

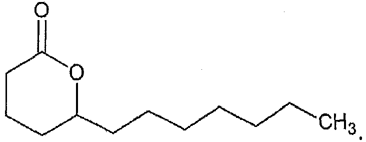

12. The flavoring agent of claim 1, wherein the flavoring agent is a flavoring agent for food or smoking articles.

13. A composition comprising the flavoring agent of claim 1.

14. The composition of claim 13,
wherein the composition further comprises a carrier acceptable for food or smoking articles, additives, or both thereof.

15. A smoking article comprising the composition of claim 13.

## Patentansprüche

1. Aromastoff, bei dem es sich um eine Verbindung, dargestellt durch die folgende Formel 1, handelt: 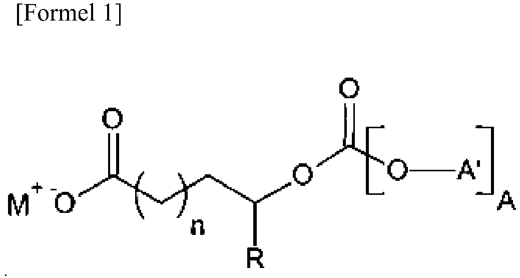
wobei in Formel 1 n eine ganze Zahl von 1 oder 2 ist, M aus Alkalimetallen und Übergangsmetallen ausgewählt ist, R eine geradkettige oder verzweigte Alkylgruppe mit 1 bis 30 Kohlenstoffatomen ist und die Einheit A eine Einheit ist, die von einer Aromaverbindung abgeleitet ist, die mindestens eines aus einem aromatischen Ring, einem aliphatischen Ring und einer aliphatischen Kette mit einer Hydroxylgruppe aufweist,
wobei die Hydroxylgruppe an einer Carbonat-Verbindungsgruppe ( 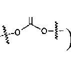 ) beteiligt ist, und A' einer Aromaverbindung mit Ausnahme der Hydroxylgruppe entspricht.

2. Aromastoff nach Anspruch 1, wobei die Aromaverbindung aus einer cyclischen Monoterpenverbindung mit einer Hydroxylgruppe, einer acyclischen Monoterpenverbindung mit einer Hydroxylgruppe, einer aromatischen C₆-C₁₀-Verbindung mit einer Hydroxylgruppe und einem nichtaromatischen C₅-C₆-Ring mit einer Hydroxylgruppe ausgewählt ist.

3. Aromastoff nach Anspruch 1, wobei die Aromaverbindung aus den folgenden Formeln ausgewählt ist:

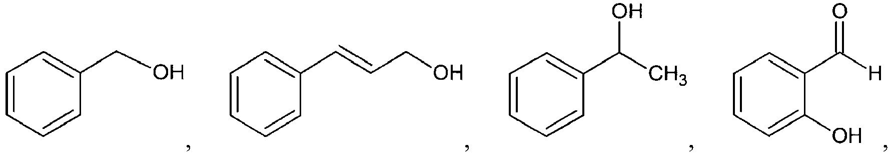

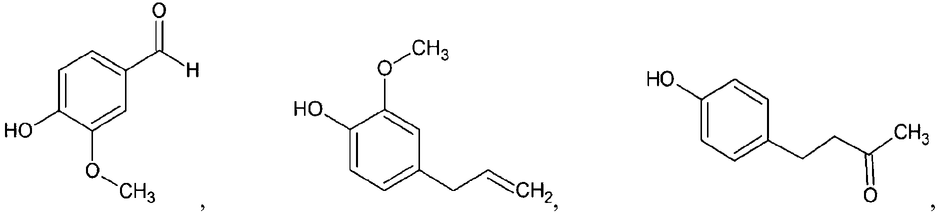

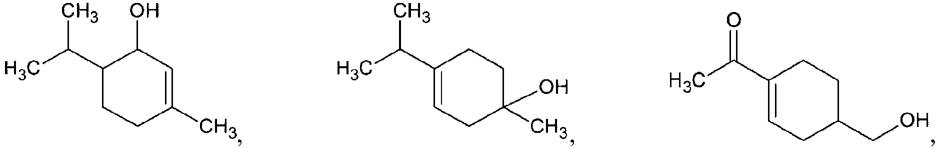

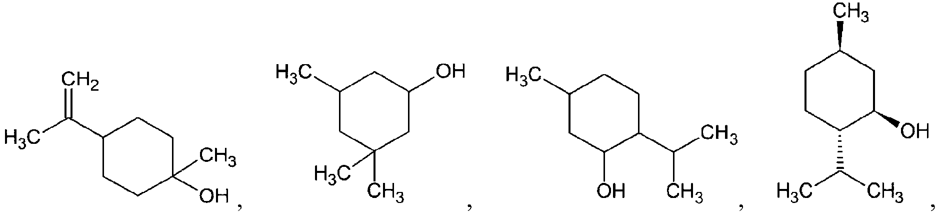

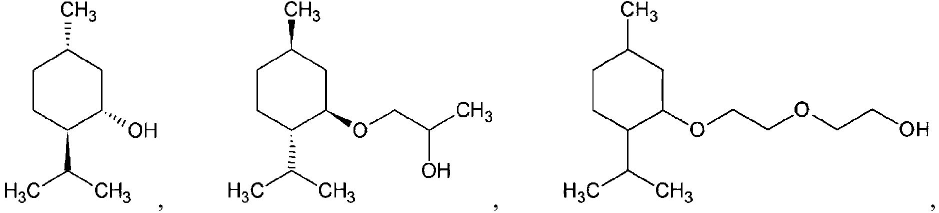

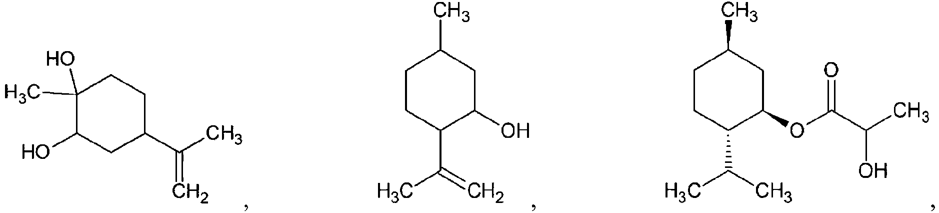

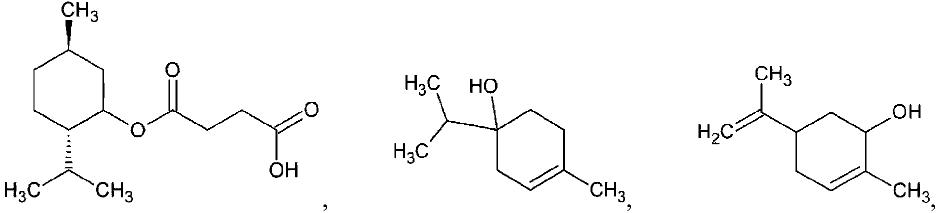

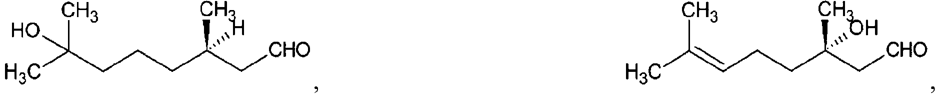

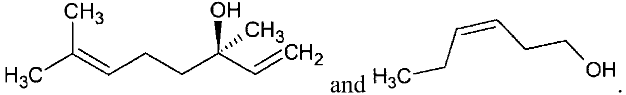

4. Aromastoff nach Anspruch 1, wobei A' aus den folgenden Formeln ausgewählt ist, wobei
* die Bindungsstelle von Sauerstoff im Carbonat ist:

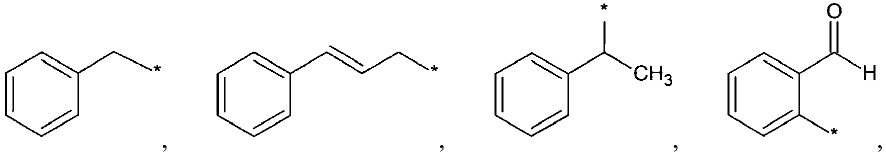

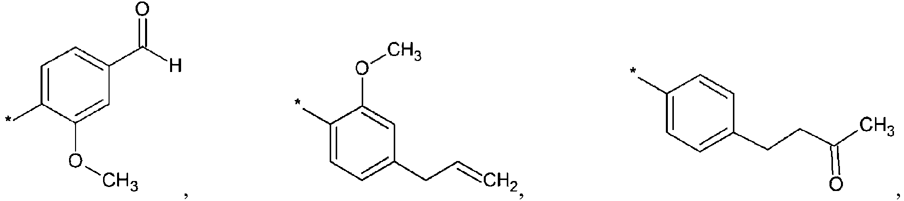

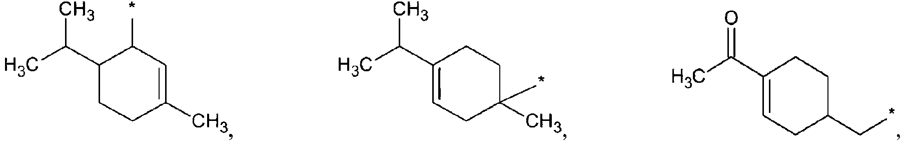

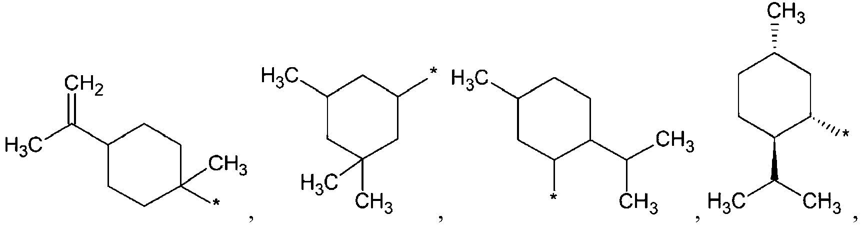

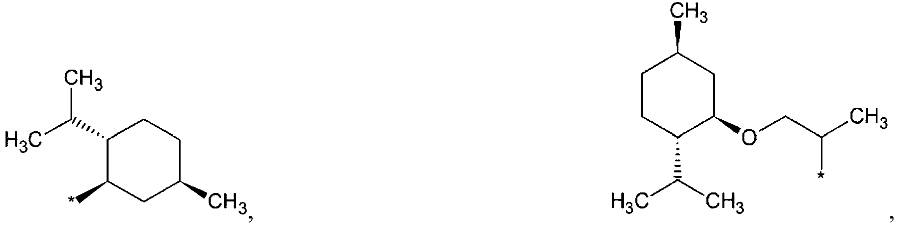

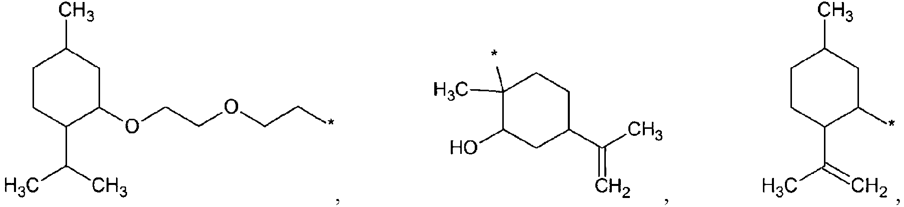

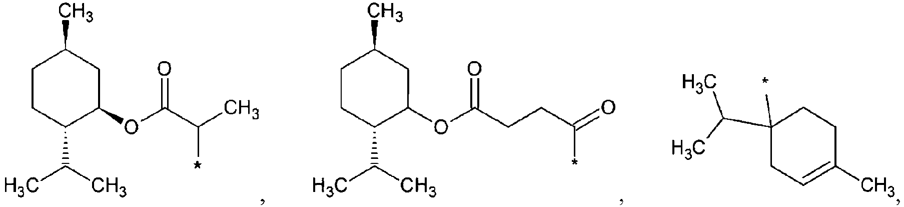

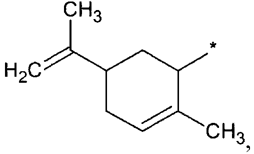

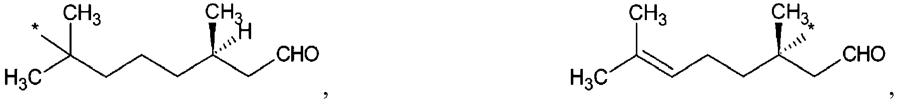

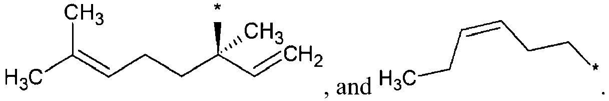

5. Aromastoff nach Anspruch 1, wobei das Übergangsmetall aus Zr, Mg, Ca, Co, Rh, Ir, Nb, Pd, Pt, Fe, Ru, Os, Cr, Mo, W, Mn, Tc, Re, Cu, Ag und Au ausgewählt ist und das Alkalimetall aus Li, Na, K, Rb und Cs ausgewählt ist.

6. Aromastoff nach Anspruch 1, wobei die Verbindung aus den folgenden Formeln 1-1 bis 1-28 ausgewählt ist: 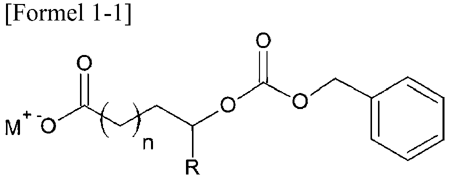 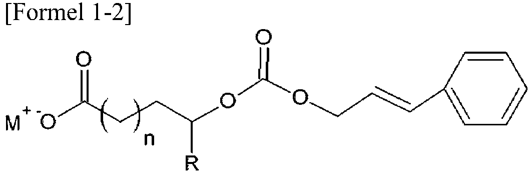 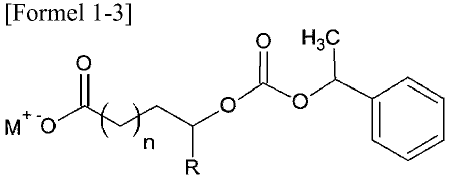 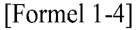 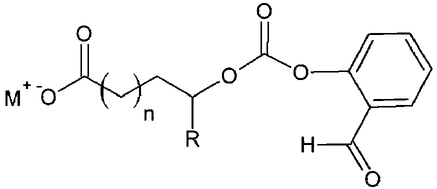 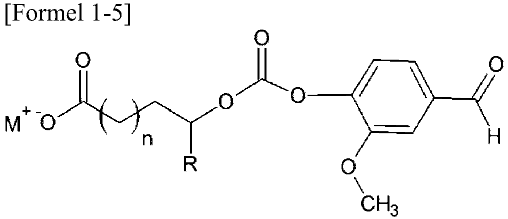 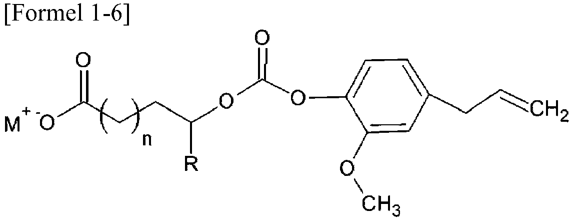 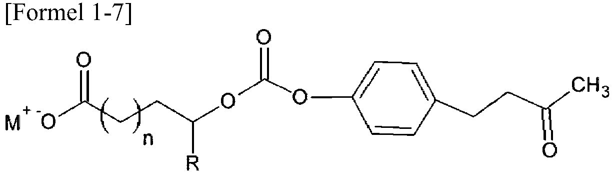 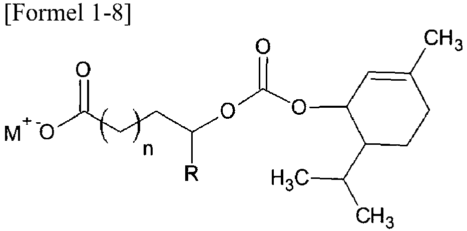 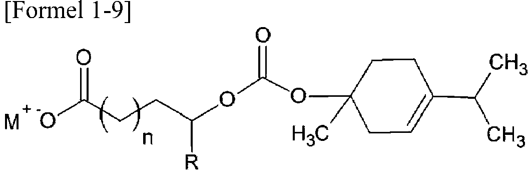 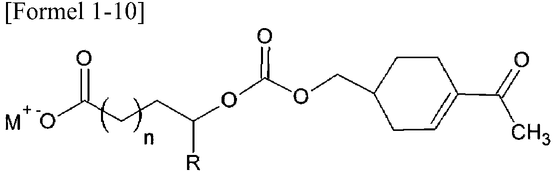 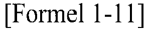 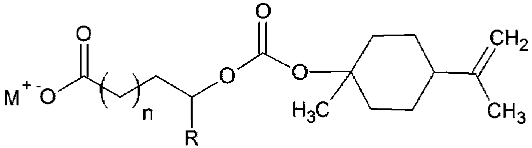 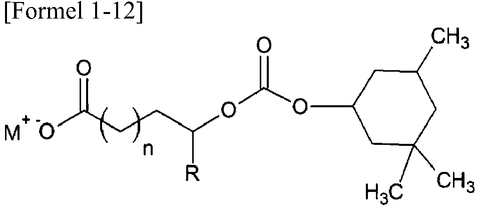 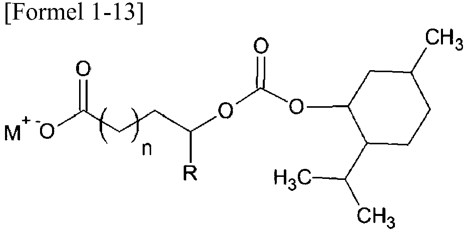 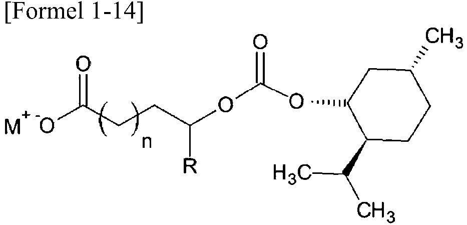 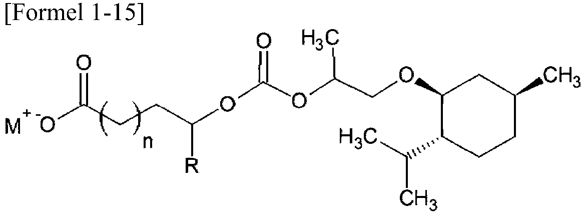 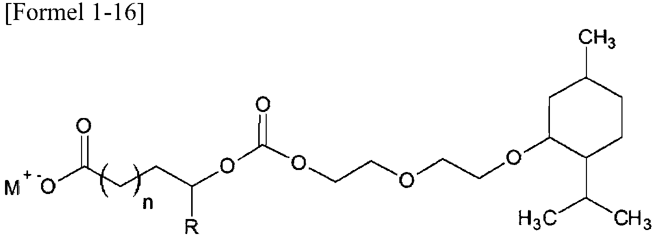 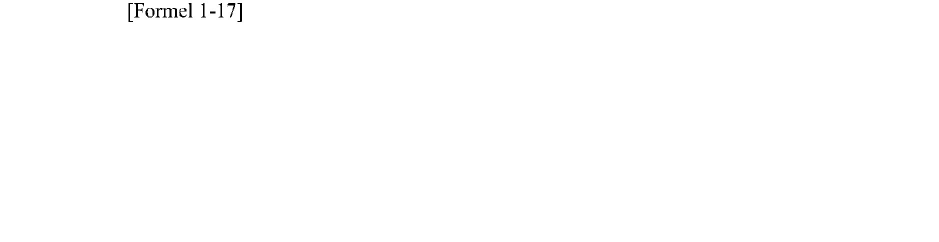 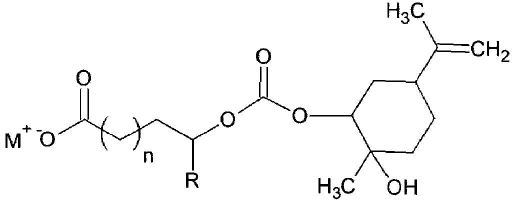 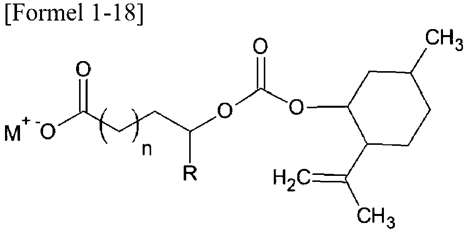 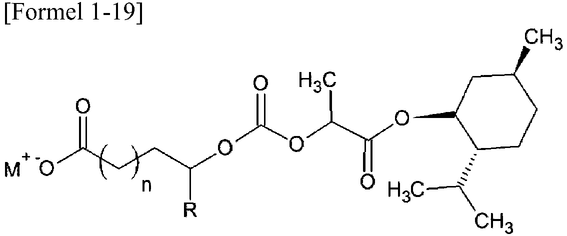 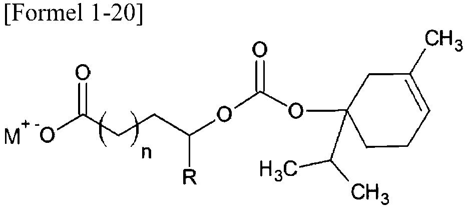 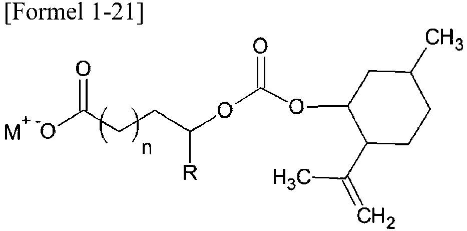 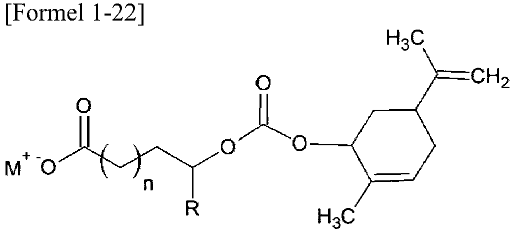 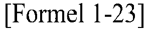 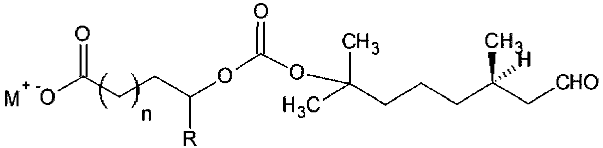 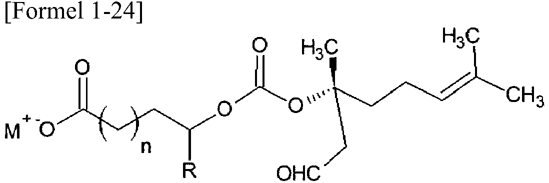 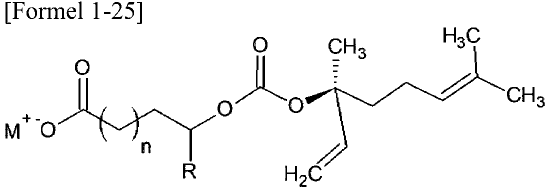 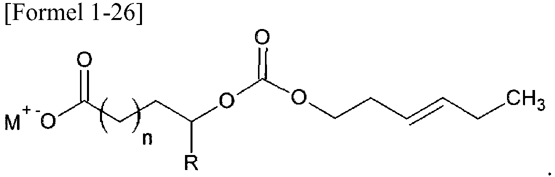 wobei M und R wie in Formel 1 oben definiert sind.

7. Aromastoff nach Anspruch 1, wobei der Aromastoff ein Aromastoff ist, der bei thermischer Zersetzung Aroma entwickelt.

8. Aromastoff nach Anspruch 1, wobei der Aromastoff ein Aromastoff ist, der sich bei thermischer Zersetzung in den Aromastoff, eine Lactonverbindung und Kohlendioxid zersetzt.

9. Aromastoff nach Anspruch 1, wobei die Verbindung eine Verbindung ist, die bei einer Temperatur von 80°C oder höher thermisch zersetzt wird.

10. Aromastoff nach Anspruch 8, wobei die Lactonverbindung ein Gamma-Lacton der folgenden Formel 2 oder ein Delta-Lacton der folgenden Formel 3 ist: 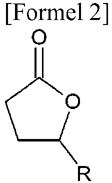 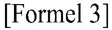 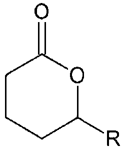 wobei R eine geradkettige oder verzweigte Alkylgruppe mit 1 bis 30 Kohlenstoffatomen ist.

11. Aromastoff nach Anspruch 8, wobei die Lactonverbindung aus den folgenden Formeln ausgewählt ist: 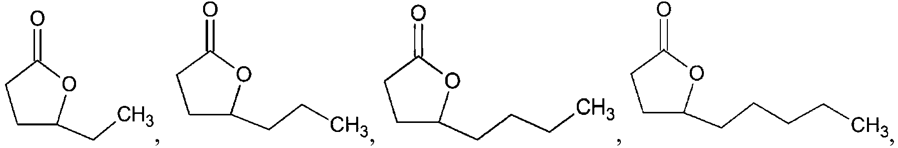 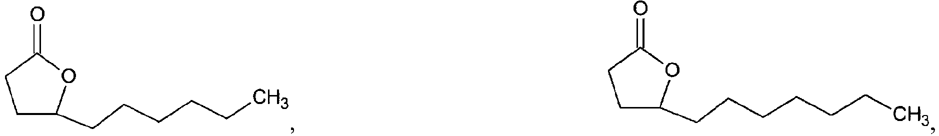 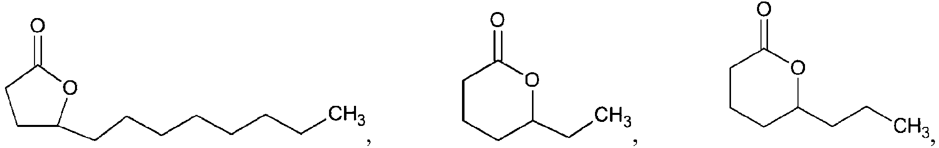 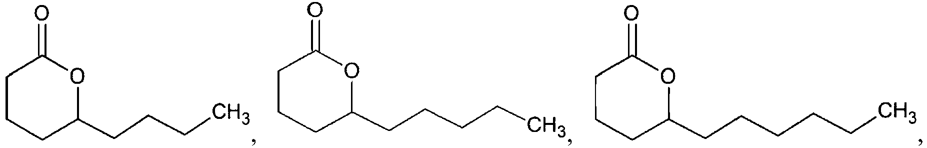 and

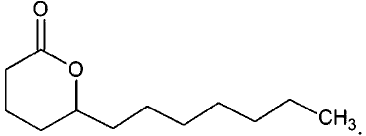

12. Aromastoff nach Anspruch 1, wobei der Aromastoff ein Aromastoff für Lebensmittel oder Rauchwaren ist.

13. Zusammensetzung, umfassend den Aromastoff nach Anspruch 1.

14. Zusammensetzung nach Anspruch 13, wobei die Zusammensetzung ferner einen für Lebensmittel oder Rauchwaren akzeptablen Träger, Zusatzstoffe oder beides umfasst.

15. Rauchartikel, umfassend die Zusammensetzung nach Anspruch 13.

## Revendications

1. Agent aromatisant, qui est un composé représenté par la Formule 1 suivante : 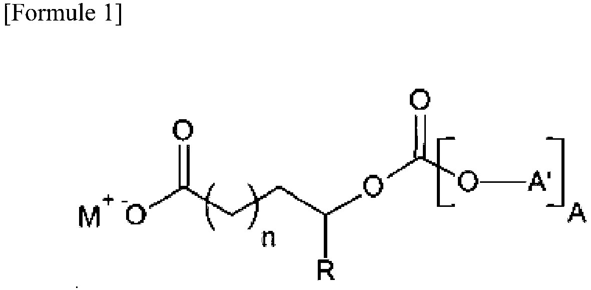 dans lequel, dans la Formule 1, n est un entier égal à 1 ou 2, M est choisi parmi des métaux alcalins et des métaux de transition, R est un groupe alkyle à chaîne droite ou à chaîne ramifiée possédant 1 à 30 atomes de carbone, et le groupement A est un groupement dérivé d'un composé aromatisant ayant au moins un élément parmi un cycle aromatique, un cycle aliphatique et une chaîne aliphatique qui ont un groupe hydroxyle, dans lequel le groupe hydroxyle participe à un groupe de liaison carbonate ( 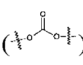 ), et A' correspond à un composé aromatisant à l'exception du groupe hydroxyle.

2. Agent aromatisant selon la revendication 1, dans lequel le composé aromatisant est choisi parmi un composé à base de monoterpène cyclique ayant un groupe hydroxyle, un composé acyclique à base de monoterpène ayant un groupe hydroxyle, un composé aromatique en C₆ à C₁₀ ayant un groupe hydroxyle et un cycle non aromatique en C₅ à C₆ ayant un groupe hydroxyle.

3. Agent aromatisant selon la revendication 1, dans lequel le composé aromatisant est choisi parmi les formules suivantes : 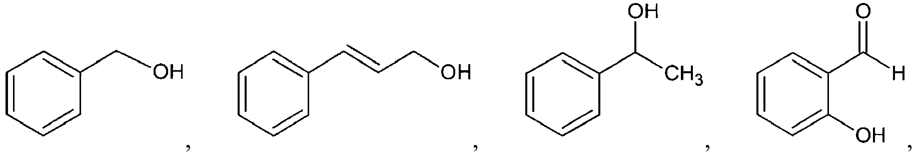 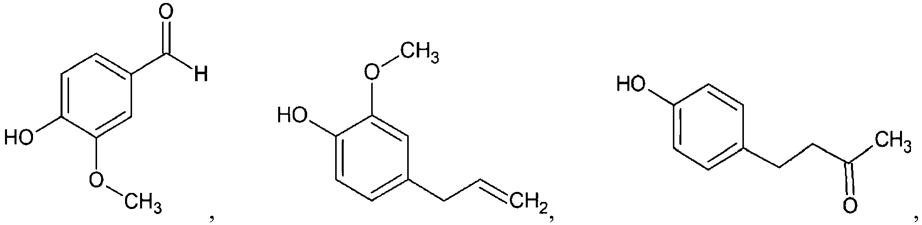 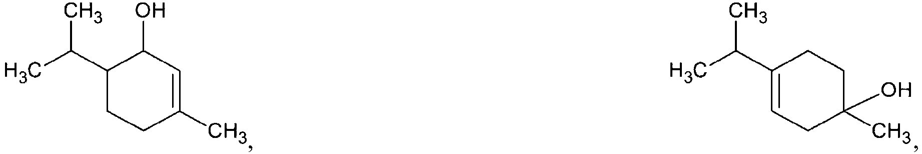 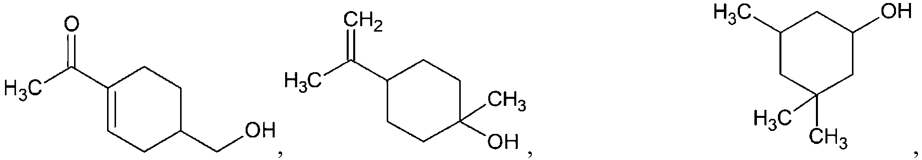 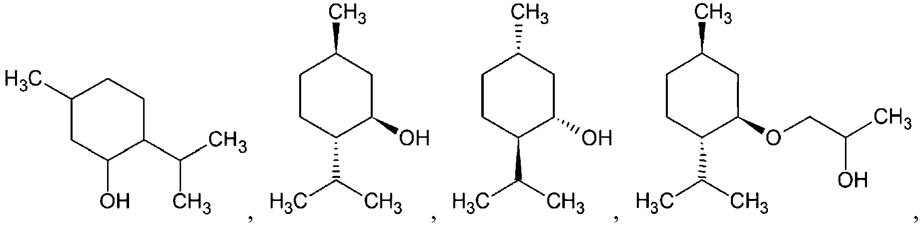 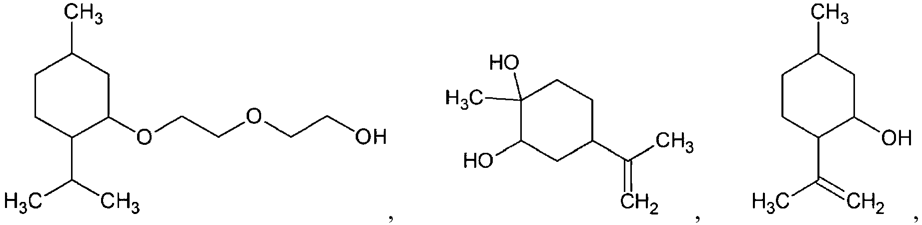 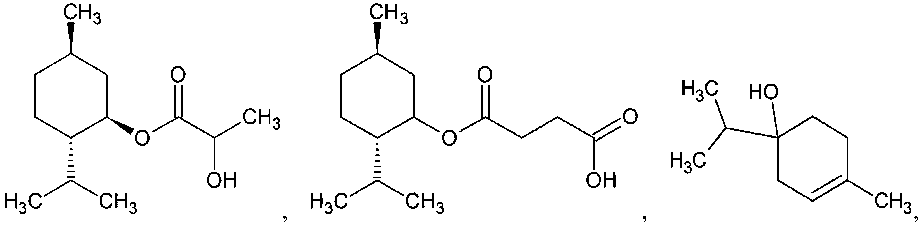 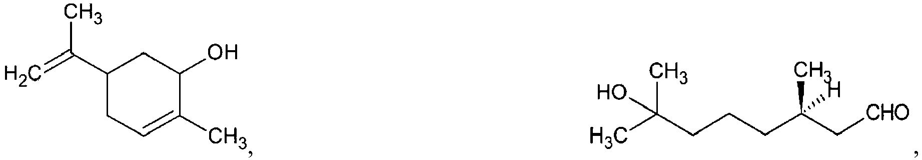 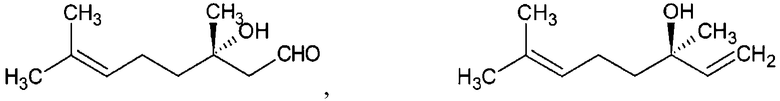 et

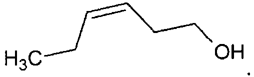

4. Agent aromatisant selon la revendication 1, dans lequel A' est choisi parmi les formules suivantes, dans lesquelles * est le site de liaison de l'oxygène dans le carbonate :

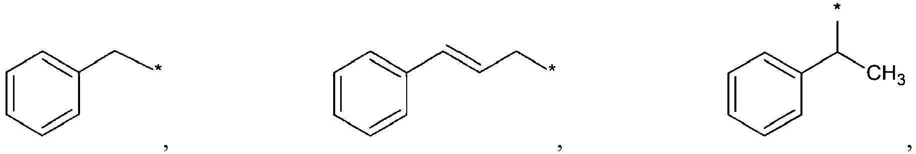

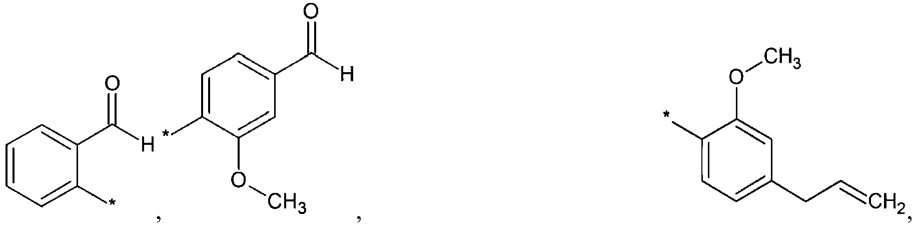

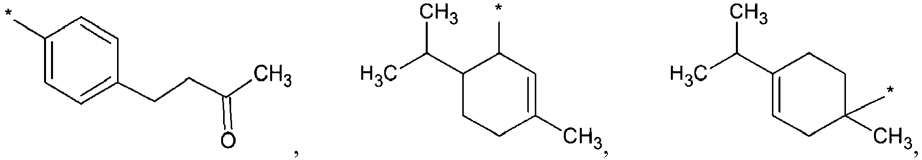

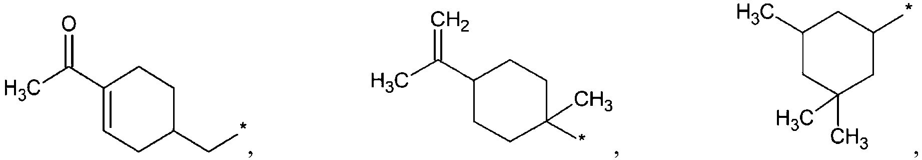

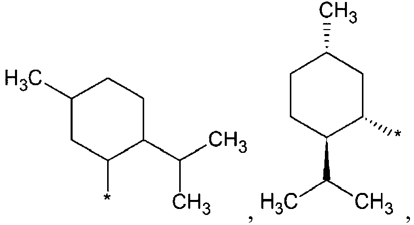

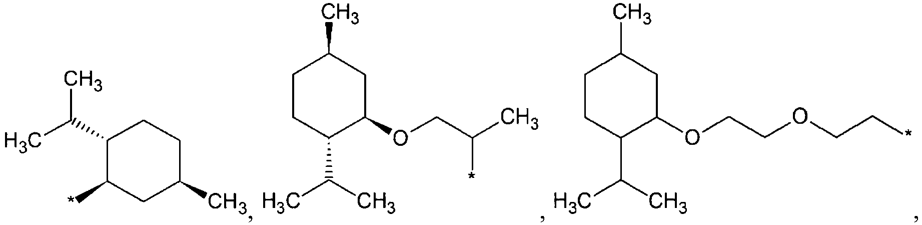

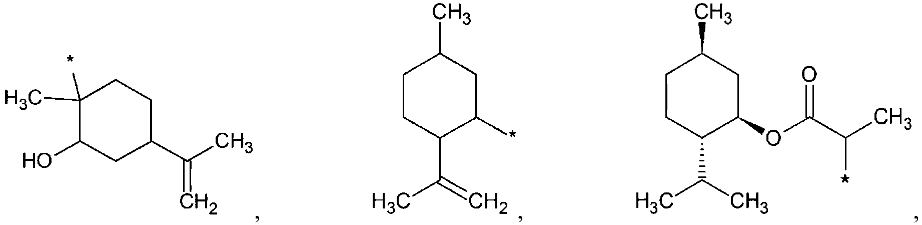

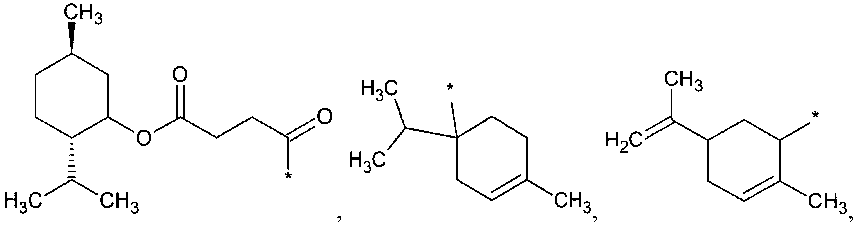

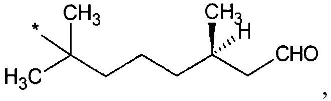

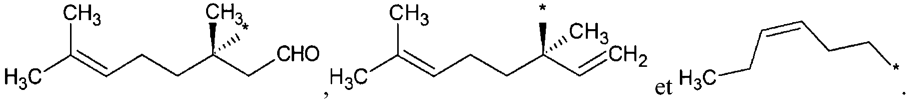

5. Agent aromatisant selon la revendication 1, dans lequel le métal de transition est choisi parmi Zr, Mg, Ca, Co, Rh, Ir, Nb, Pd, Pt, Fe, Ru, Os, Cr, Mo, W, Mn, Tc, Re, Cu, Ag et Au, et le métal alcalin est choisi parmi Li, Na, K, Rb et Cs.

6. Agent aromatisant selon la revendication 1, dans lequel le composé est choisi parmi les Formules 1-1 à 1-28 suivantes : 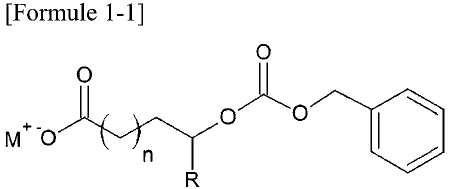 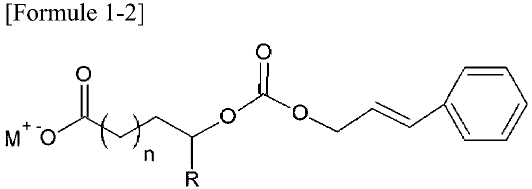 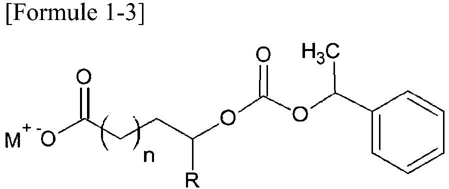 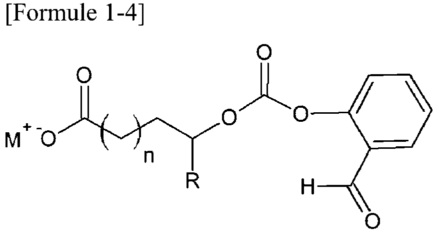 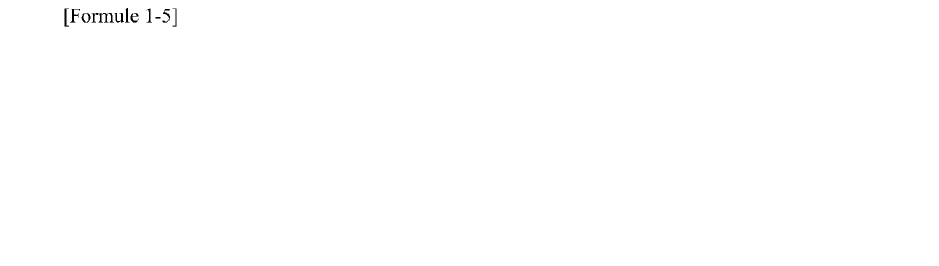 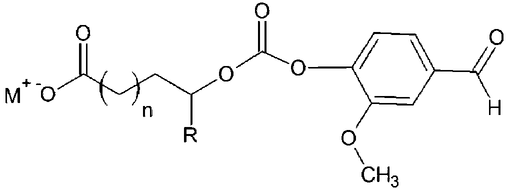 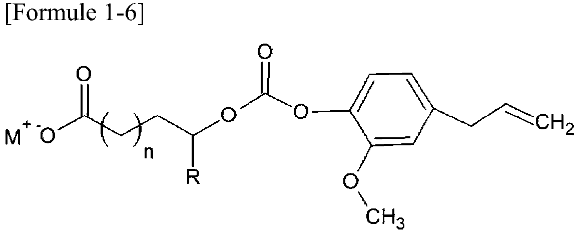 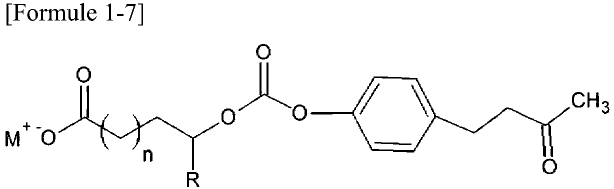 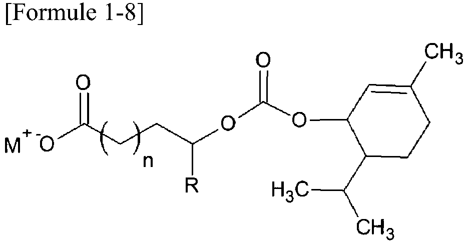 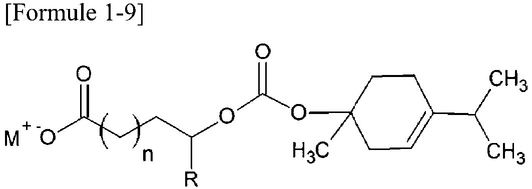 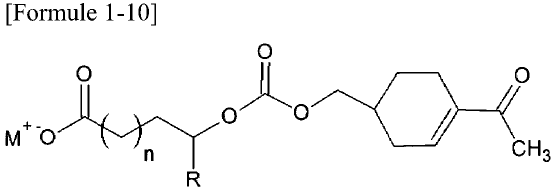 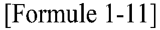 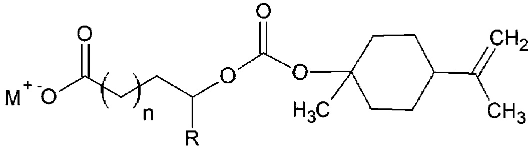 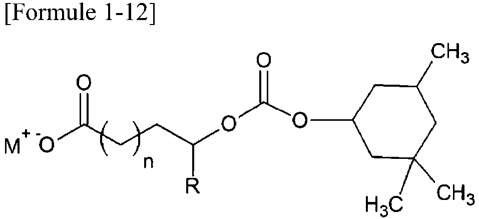 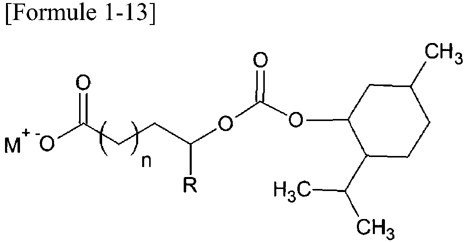 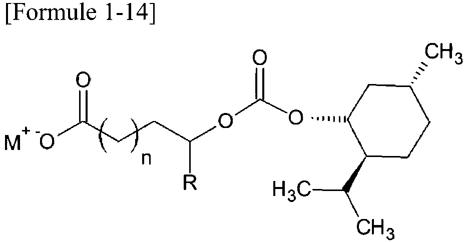 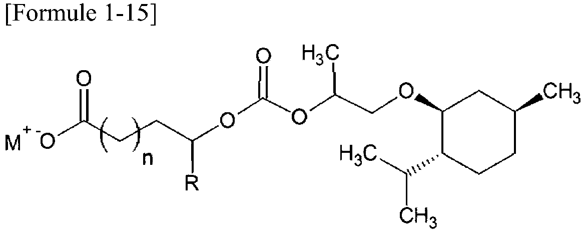 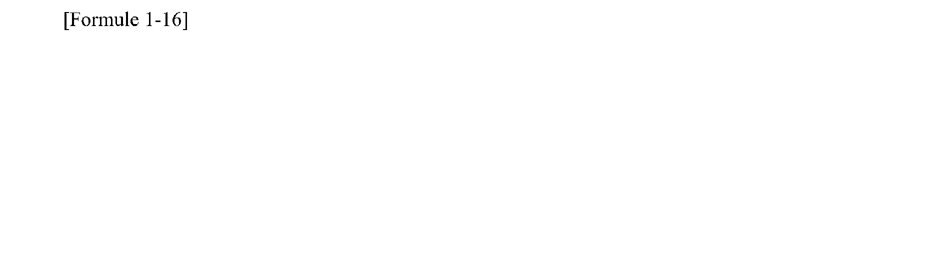 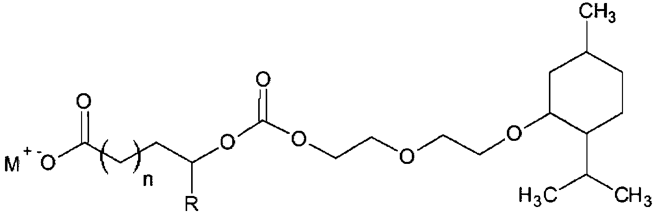 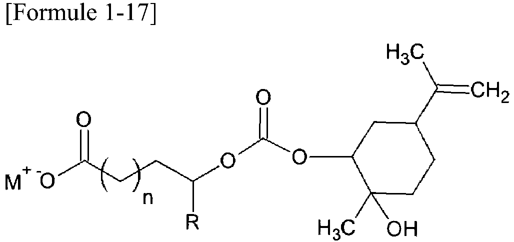 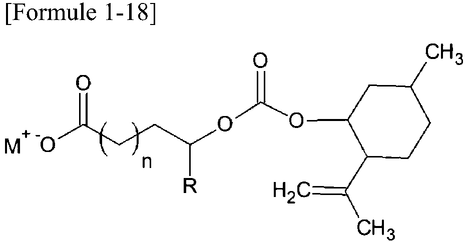 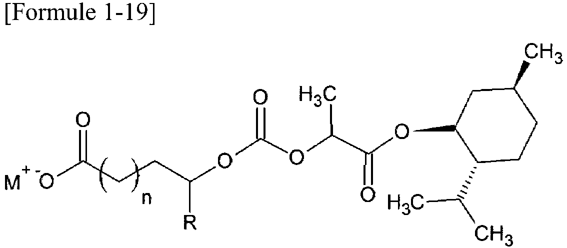 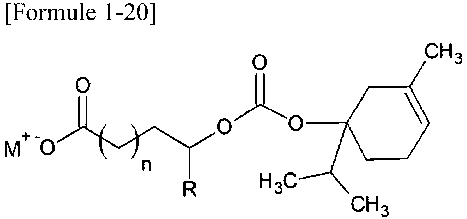 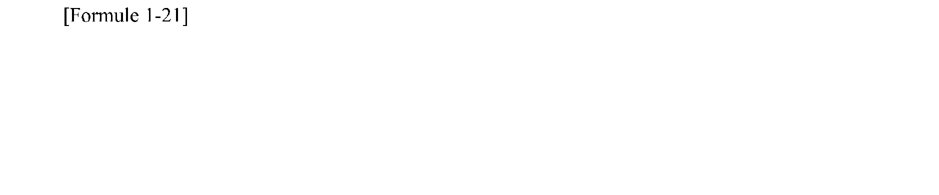 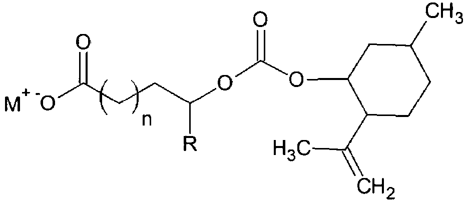 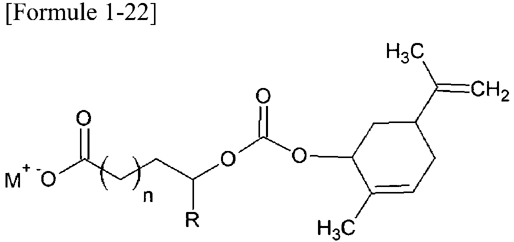 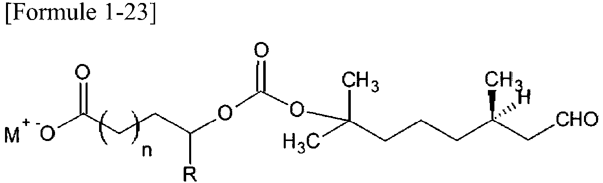 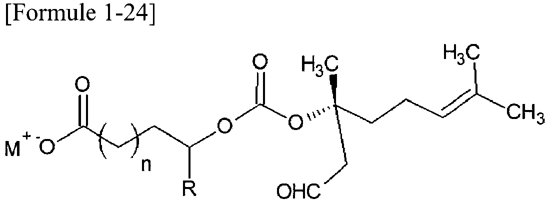 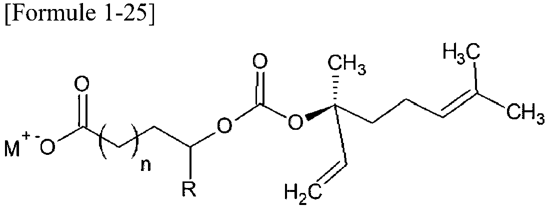 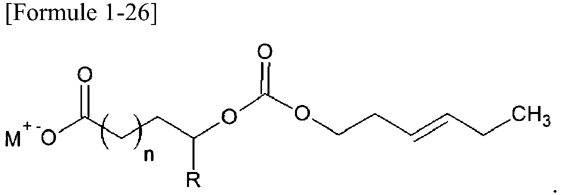 dans lesquelles M et R sont tel que défini dans la Formule 1 ci-dessus.

7. Agent aromatisant selon la revendication 1, dans lequel l'agent aromatisant est un composé d'agent aromatisant qui développe l'arôme lors d'une décomposition thermique.

8. Agent aromatisant selon la revendication 1, dans lequel l'agent aromatisant est un agent aromatisant qui se décompose en composé aromatisant, en composé lactone et en dioxyde de carbone pendant la décomposition thermique.

9. Agent aromatisant selon la revendication 1, dans lequel le composé est un composé qui se décompose thermiquement à une température de 80 °C ou à une température supérieure.

10. Agent aromatisant selon la revendication 8, dans lequel le composé lactone est la gamma lactone ayant la Formule 2 suivante ou la delta lactone ayant la Formule 3 suivante : 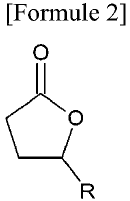 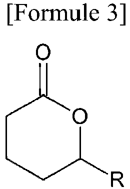 dans lesquelles R est un groupe alkyle à chaîne droite ou à chaîne ramifiée possédant 1 à 30 atomes de carbone.

11. Agent aromatisant selon la revendication 8, dans lequel le composé lactone est choisi parmi les formules suivantes : 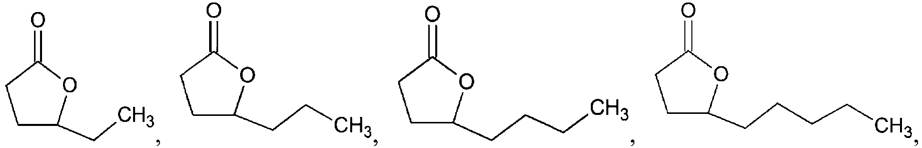 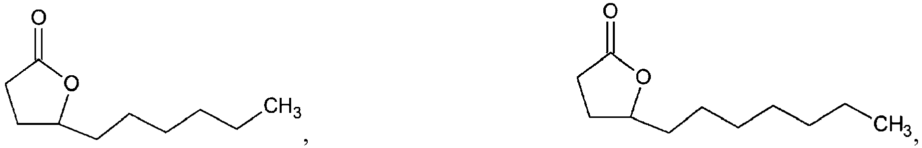 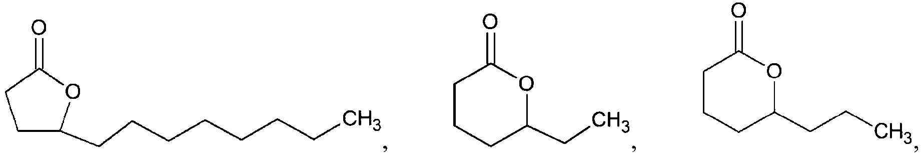 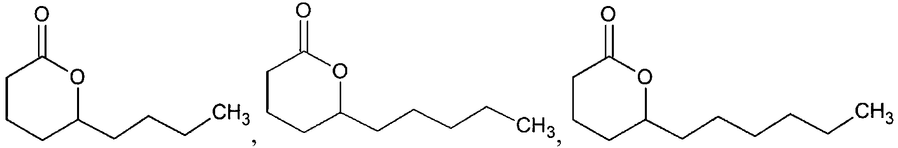 et

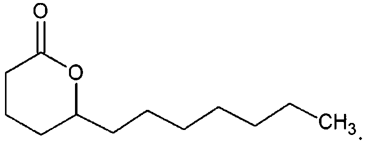

12. Agent aromatisant selon la revendication 1, dans lequel l'agent aromatisant est un agent aromatisant pour des aliments ou des articles à fumer.

13. Composition comportant l'agent aromatisant de la revendication 1.

14. Composition selon la revendication 13,
dans laquelle la composition comporte en outre un support acceptable pour des aliments ou des articles à fumer, des additifs, ou les deux.

15. Article à fumer comportant la composition selon la revendication 13.
